(19) Europäisches Patentamt
European Patent Office
Office européen des brevets

(11) Publication number: **0 366 451 B1**

# (12) EUROPEAN PATENT SPECIFICATION

(45) Date of publication of patent specification: **06.04.94**

(51) Int. Cl.$^5$: **A61K 31/13**, C07C 217/40

(21) Application number: **89311013.0**

(22) Date of filing: **25.10.89**

## (54) The use of oxidized polyamines, especially NN'-Bis-(3-propionaldehyde)-1-4-diaminobutane (sperminedialdehyde) as immunosuppressive agents.

(30) Priority: **26.10.88 US 262760**

(43) Date of publication of application:
**02.05.90 Bulletin 90/18**

(45) Publication of the grant of the patent:
**06.04.94 Bulletin 94/14**

(84) Designated Contracting States:
**AT BE CH DE ES FR GB GR IT LI LU NL SE**

(56) References cited:
**DE-A- 2 908 032**

**BR. J CANCER, vol. 39, 1978, pages 548-557; J. M. GAUGAS et al.: "Evidence for serum binding of oxidized spermine and its potent G1-phase inhibition of cell proliferation"**

**EUR. J. IMMUNOL., vol. 11, no. 3, 1981, pages 266-269; R. S. LABIB et al.: "Exzymatic oxidation of polyamines. Relationship to immunosuppressive properties"**

**ISREAL J. MED. SCI., vol. 1, no. 4, 1965, pages 541-551; U. BACHRACH et al.: "Antiphage action of oxidized polyamines"**

**CHEMICAL ABSTRACTS, vol. 75, no. 19, 8th November 1971, page 190, abstract no. 117960c, Columbus, Ohio, US; & JP-A-71 24 365 (TAKEDA CHEMICAL INDUSTRIES, LTD) 13-06-1971**

(73) Proprietor: **ORTHO PHARMACEUTICAL CORPORATION**
**U.S. Route 202**
**P.O. Box 300**
**Raritan New Jersey 08869-0602(US)**

(72) Inventor: **Lau, Catherine Y.**
**1 Decourcy Court**
**Unionville Ontario, M3L 246(CA)**

(74) Representative: **Fisher, Adrian John et al**
**CARPMAELS & RANSFORD**
**43 Bloomsbury Square**
**London WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention relates to the use of oxidized polyamines, and especially aminoaldehydes such as NN′-Bis-(3-propionaldehyde)-1-4-diaminobutane (spermine bisaldehyde) both in vitro and in vivo to elicit an immunosuppressive response in living cells. It also relates to the therapeutic application of these compounds to induce an immunosuppressive response in a living organism.

Polyamines such as spermine, spermidine and putrescine are widely distributed in mammalian cells, although they are found to differ in their relative concentrations. Oxidized Polyamines are believed to inhibit growth of parasites (D.M.L. MORGAN and J.R. CHRISTENSEN, Adv. Polyamine Res., 4, 169-174 (1983); D.M.L. MORGAN, U. BACHRACH, Y.G. ASSARAF, E. HARARI and J. GOLENSER, Biochem. J., 236, 97-101 (1986)), suppress infectivity of selected strains of phage and bacteria (U. BACHRACH, S. DON and H. WIENER, J. Gen. Virol., 13(Pt. 3), 415-22 (1971); K. NISHIMURA, T. KOMANO and H. YAMADA, Biochim. Biophys. Acta, 247(1), 153-6 (1971) J.G. HIRSCH and R.J. DUBOS, J. Exp. Med., 95, 919 (1952); C.W. TABOR and S.M. ROSENTHAL, J. Pharmacol., 116, 139 (1956)) and inactivate several strains of viruses (U. BACHRACH and E. ROSENKOVITCH, Appl. Microbiol., 23(2), 232-5 (1972); U. BACHRACH and S. DON, J. Gen. Virol., 11(Pt. 1), 1-9 (1971); U. BACHRACH, C.W. TABOR and H. TABOR, Biochem. Biophys. Acta, 78, 768 (1963); U. BACHRACH and J. LEIBOVICI, Isr. J. Med. Sci., 1, 541 (1965); J. SCHINDLER, Experientia, 21, 697 (1965); E. KATZ, T. GOLDBLUM, U. BACHRACH and N. GOLDBLUM, Isr. J. Med. Sci., 3, 575 (1967)). The literature does not make clear however, whether the inhibitory effect is due to aminoaldehydes or other toxic side products such as hydrogen hydroxide and ammonia released during the oxidation of spermine by PAO.

Hence, despite the impressive list of studies performed with oxidized spermine, the actual molecular structure(s) mediating the specific immunosuppressive or inhibitory activities is unknown, Oxidation of spermine by PAO revealed six major oxidation products in addition to ammonia and hydrogen peroxide (R.S. LABIB and T.B. TOMASI, JR., Eur. J. Immunol., 11, 266-269 (1981)). The inhibitory effect of each of the Products has not been analyzed but it was believed that the dioxidized spermine or spermine dialdehyde (NN′-Bis-(3-propionaldehyde)-1-4-diaminobutane) would demonstrate activity. Israel et al, Supra, synthesized this molecule together with another analogue and found that both compounds exhibited cell inhibitory activities in vitro. These investigations however failed to find significant in vivo efficacy with the dialdehydes they synthesized. Furthermore, these molecules were quite toxic, spermine dialdehyde (NN′-Bis-(3-propionaldehyde)-1-4- diaminobutane) showed severe acute toxicity with an $^{LD}100$ at 40 mg/kg when given intraperitoneally.

### Brief Description of the Drawings

#### Figure 1

Human T & B lymphocytes were incubated separately with different concentrations of spermine dialdehyde in vitro for 1 hour. Cells were washed extensively and set up in a PHA (for T cell) or PWM (B cell) proliferation assay. A graph is depicted wherein percentage of response is plotted as a function of concentration of spermine dialdehyde.

#### Figure 2

Mouse spleen and bone marrow cells were incubated separately with different concentrations of spermine dialdehyde in vitro for 1 hour. Cells were washed extensively and set up in a Con A stimulated proliferation assay. A graph is depicted wherein percentage response is plotted as a function of various concentrations of spermine dialdehyde.

#### Figure 3

C57BL spleen and bone marrow cell mixtures were treated with different concentrations of spermine dialdehyde or control preparation and injected i.v. into lethally irradiated AKR mice. A graph is depicted wherein percentage survival is scored as a function of time.

Figure 4

(LewXBN) rats were injected i.v. daily for 8 days with different doses of spermine dialdehyde. On day 2, BN rat spleen cells were injected subcutaneously into the left foot pads of the (LewxBN)F1 rats. These rats were sacrificed in 7 days and weights of both left and right popliteal lymph nodes were removed. The difference in weight is plotted as a function of spermine dialdehyde concentrations used.

Figure 5

C57BL mice were injected i.p. daily with different doses of spermine dialdehyde. On day 2, they also received 107 DBA spleen cells. Seven days later, animals were sacrificed and percentage of cytotoxic T cells (measured by % specific lipes generated in the spleen was calculated and plotted as a function of concentration of spermine dialdehyde.

Figure 6

In a skin graft transplant experiment, BAH/C ($H2^d$) skin grafts were transplanted into C3H mice. Spermine dialdehyde was administered S.C. right after transplantation, and continued throughout the study. Cyclosporin A was run as a positive control. % skin graft survival was plotted versus days post transplantation.

Figure 7

Delayed type hypersensitivity was induced in the footpad of B6D2F1 mice and swelling measured in mm. Spermine dialdehyde was administered i.p. and i.m. at various timeperiods.

Figure 8

Effect of SDA and its analogues on Concanavalin A-stimulated splenic T cell proliferation.

Splenic cells were treated with various concentrations of SDA or an analogue for 30 min. at 37 ° C. Cells were washed and cultured in the presence of Concanavalin A for 3 days. [$^3$H] Thymidine 0.1μCi/well was added and cells were harvested after 18-24 hrs. SDA was the most potent antiproliferation drug, with an $IC_{50}$ of 0.70μM. Although butyl dialdehyde was 30 fold less potent than SDA, it should be metabolically more stable and hence clinically more relevant.

Figure 9

Inhibition of S-Adenosylmethionine Decarboxylase (AdoMet DC) by SDA and its Analogue.

Murine leukemic L1210 cell supernatants were incubated with different concentrations of SDA or butyl dialdehyde, and AdoMet DC activity was estimated by monitoring the release of [$^{14}$C] $CO_2$. Inhibition curves for both drugs were superimposable with $IC_{50}$ values of 0.12-0.13 x $10^{-3}$ M.

Figure 10

Regulation of polyamine biosynthetic enzymes by SDA and other known inhibitors.

The figure illustrates the comparative profile of the inhibition of key regulatory enzymes of polyamine synthesis by SDA, its analogue, butyl dialdehyde (BDA), difluoromethyl orinithine (DFMO) and methyl glyoxal bis (guanylhydrazone) (MGBG). DFMO inhibited ODC with high potency, while MGBG selectively reduced AdoMet IC activity. The analogue BDA was similar to MGBG in its selectivity to AdoMet DC. However, SDA was the only drug that inhibited both of the key enzymes, thereby eliminating the compensatory response (of DFMO on AdoMet DC) and ensuring complete inhibition of polyamine biosynthesis.

The present invention provides an in vitro method for inducing an immunosuppressive response in living cells which comprises the administration to those cells of an effective amount of a substantially pure form of a compound having the General Formula:

OCH - $ALK^1$ - $NR^2$ - $CH_2$ - $AlK^2$ - $CH_2$ -$NR^2$ - Z

wherein

$ALK^1$ is independently alkylene;
$R^2$ is independently hydrogen or -$CH_2R^3$;
$R^3$ is independently alkyl;
$ALK^2$ is alkylene;
Z is H or $ALK^1$-CHO;

or the acid addition salt thereof.

[ General Formula I ]

This immunosuppressive response is particularly selective for the suppression of the proliferation of T cell populations, especially the helper T cell and cytotoxic T cell subpopulations.

Also provided are methods for manufacturing a medicament for inducing an immunosuppressive response in a living organism which comprises the administration of a compound having the above formula to said organism, in substantially pure form and in an amount effective to induce said response.

The methods as described herein are particularly suitable in the therapeutic control of various immunologically related disease states such as graft vs. host rejections, delayed hypersensitivity and the like.

The present inventors have discovered that compounds, particularly those obtained by synthetic methods, having the General Formula:

OCH - $ALK^1$ - $NR^2$ - $CH_2$ - $AlK^2$ - $CH_2$ - $NR^2$ - Z

wherein

$ALK^1$ is independently alkylene;
$R^2$ is independently hydrogen or -$CH_2R^3$;
$R^3$ is independently alkyl;
$ALK^2$ is alkylene;
Z is H or $ALK^1$-CHO;

or the acid addition salt thereof.

[ General Formula I ]

are useful in inducing an immunosuppressive response in living cells when applied to those cells in substantially pure form. Particularly useful are compounds of General Formula I wherein $ALK^1$ $ALK^2$, and $R^3$ are each independently straight or branched chain alkylene of 1 to 8 carbons, preferably 1 to 6 carbons, more preferably 1 to 4 carbon atoms, and most preferably 1 to 2 carbon atoms. In more preferred embodiments, $R^2$ is hydrogen. In the particularly preferred embodiments, Z is $ALK^1$-$CHO_1$, $R^2$ is hydrogen, and $ALK^1$ and $ALK^2$ are each independently 1 to 6 carbon atoms, preferably 1 to 4 carbon atoms, most preferably 1 to 2 carbon atoms. Of the most preferred compounds for use herein may be mentioned spermine dialdehyde.

The presently described compounds are also useful in the therapeutic inducement of an immunosuppressive response in a living organism. In particular, one of the compounds, synthetic spermine dialdehyde, used according to the present invention has demonstrated enhanced efficacy and a substantially reduced toxicity [not lethal up to 400 mg/kg, contrary to what was reported in the literature ($LD_{100}$ 40 mg/kg, Israel (1973) Supra)]. The use of these molecules, particularly spermine dialdehyde, both in in vitro applications as well as in vivo applications to induce an immunosuppressive response is therefore described in detail herein.

As used herein, the term "immunosuppressive response" refers to a suppression in the proliferation of cell types originating from hemopoietic stem cells, particularly the lymphoid lineage. The use of the compounds as described herein is particularly suitable in the selective suppression of the T-cell population, particularly the T-cytotoxic, T-helper subpopulations. The immunosuppression may also be described as antigen nonspecific, which is exemplified by a 99% suppression of mitogen-induced T cell proliferation. In some cases, contrary to the art, this selective suppression has been shown to be irreversible.

The compounds for use as described herein may be obtained in any convenient manner. The compounds are preferably used in "substantially pure form" which means substantially free of enzymes or other agents that might be present during its formation, and that will interfere with its efficacy or that might increase toxicity. Hence, in its preferred form, the compounds are preferably 95% pure, more preferably 99% pure, and most preferably 100%, as measured by nuclear magnetic resonance, mass spectro analysis,

and HPLC. Inert materials may be present in trace amounts.

The compounds may be obtained by enzymatic oxidation of naturally occurring polyamines by techniques known to the art, as long as the product is purified from the enzymes using conventional purification means such as chromatography and the like. Suitable enzymes for use in an enzymatic oxidation process are those effective to bring about the rapid oxidative deamination of the natural polyamine. Illustrative of such enzymes are amine oxidases obtained from ruminant sera such as beef serum, sheep serum, fetal calf serum and the like. Also applicable are oxidases obtained from mouse amniotic fluid, human pregnant sera, and the like.

To avoid interference with contaminants, it is preferred that the compound be synthesized de novo, using organic synthesis techniques such as those described below. In the particularly preferred embodiments, compounds for use herein are amino dialdehydes. The dialdehyde compound is synthesized by conversion of a diacetal in the presence of an acid. Preferred diacetals are represented by Formula:

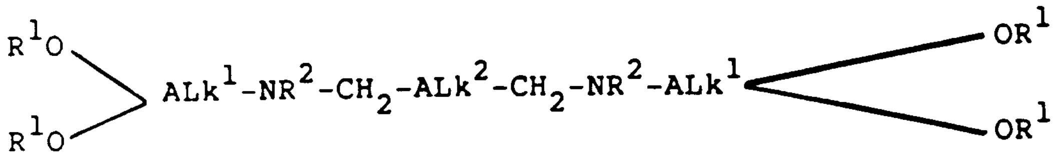

(X)

wherein

- $R^1$ is independently alkyl or benzyl
- $ALk^1$ is independently alkylene;
- $R^2$ is independently hydrogen or $-CH_2R^3$
- $R^3$ is independently alkyl; and
- $ALk^2$ is alkylene,

Preferred reactions may be exemplified by the following schemes:

REACTION SCHEME (I)

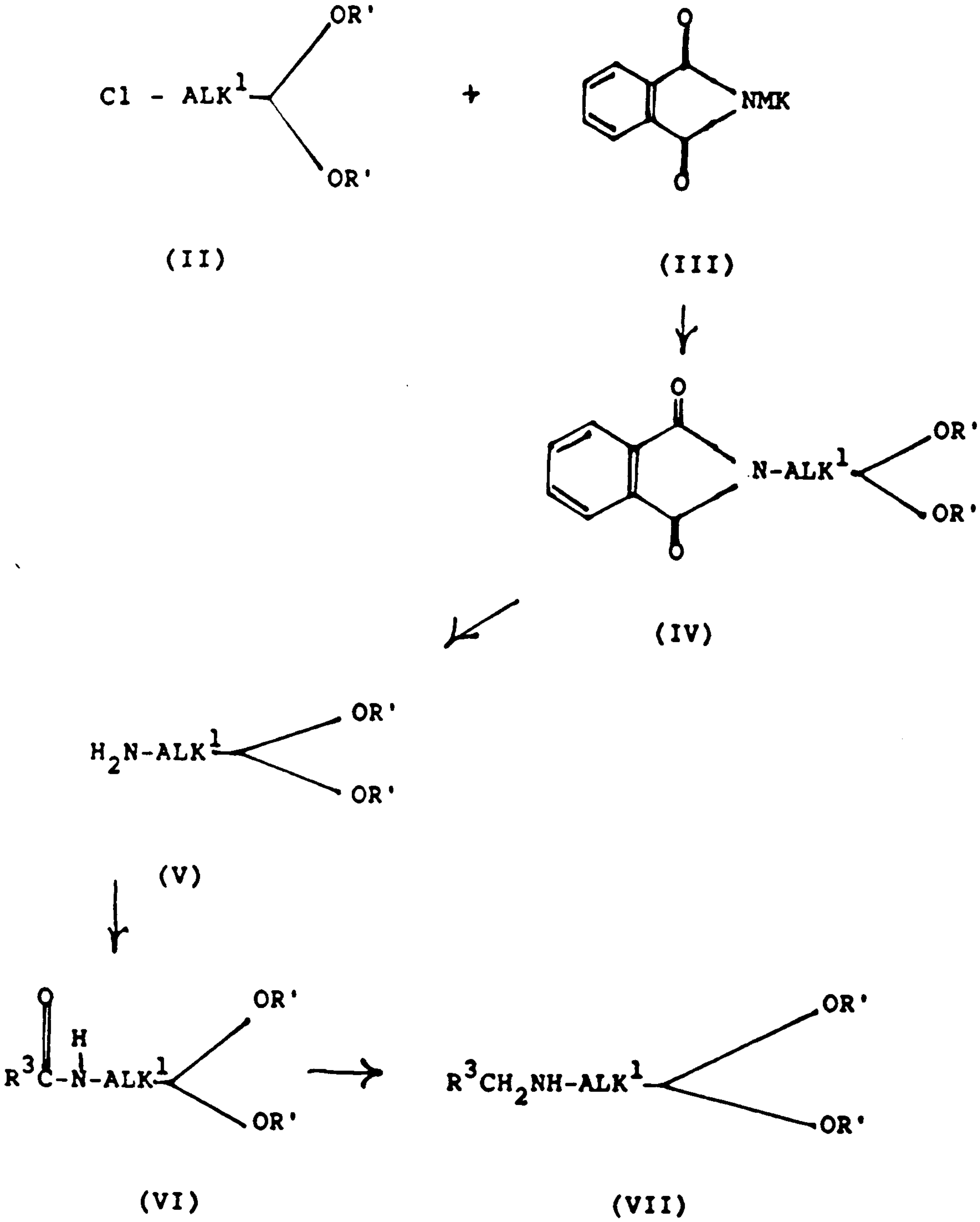

(II) (III) (IV) (V) (VI) (VII)

REACTION SCHEME (II)

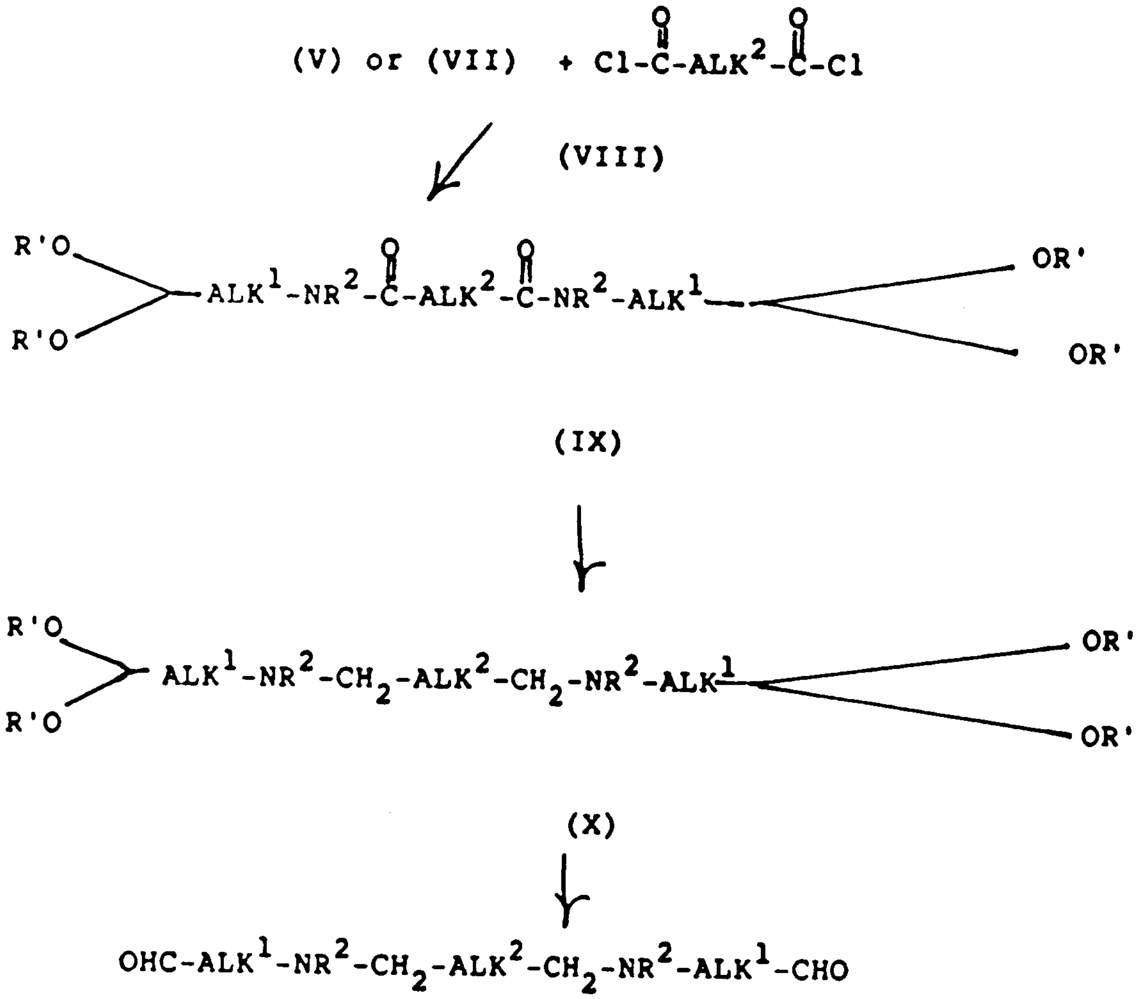

(VIII)

(IX)

(X)

(I)

The above reaction Schemes I and II set forth a summary of preferred syntheses for compounds of Formula (X) which may be stored and used to generate compounds of General Formula (I).

A chloroaldehyde of Formula (II) in acetal form is reacted with potassium phthalimide of Formula (III) in equal molar amounts at an elevated temperature to yield the phthalimide of Formula (IV). Examples of the compound of Formula (II) are 3-chloroacetaldehyde diethyl acetal and 3-chloro propionaldehyde diethylacetal. The amine is then released by reaction with hydrazine at an elevated temperature, yielding the primary amine of Formula (V). The primary amine (V) can then be taken on as shown in Reaction Scheme II or can be modified to produce a secondary amine. For the secondary amine, the primary amine of Formula (V) is reacted with an anhydride such as formic-acetic anhydride or acetic anhydride at temperatures that can vary from about 0 °C to about room temperatures to yield the amide of Formula (VI). The amide is then reduced with lithium aluminum hydride in tetrahydrofuran at reflux to yield the secondary amine of Formula (VII). In Formulae (II), (IV), (V), (VI), and (VII), $R^1$, $ALK^1$ and $R^3$ are as defined for Formula

(X) above.

To prepare the dibenzyl acetal of Formula (V) or (VII) the corresponding diethyl compound may be dissolved in a solvent and reacted with benzyl alcohol in the presence of a small amount of acid such as trichloracetic acid followed by distillation to drive off the ethanol byproduct. For a different alkyl moiety, the diethyl compound may be dissolved in the alcohol, e.g. methanol for the dimethyl acetal, and reacted as in the benzyl case. To avoid cyclization, it may be preferable to subject a compound of Formula II to this treatment, due to the presence of the acid.

In Reaction Scheme II, the primary or secondary amine, designated as compound (V) or (VII), respectively, is reacted with a diacid chloride of Formula (VIII). Examples of diacid chlorides are succinyl chloride and glutaryl chloride. The reaction is conducted at a low temperature of about -70° to -20° in the presence of a mild base such as triethylamine to yield the diamide of Formula (IX). The diamide (IX) is then reduced with a reducing agent such as lithium aluminum hydride in tetrahydrofuran at reflux to yield the diamine diacetal of Formula (X). The diamine diacetal (X) can be stored for extended periods and brought to the site of administration to a patient for the conditions described above. After reduction with lithium aluminum hydride, the final product is preferably purified by making the crystalline salt form of the diamine (X) with an acid. The free amine can then be resynthesized from the salt. However, storage may be easier with the salt form. It should be noted that creation of the salt with acid must be carried out carefully since excessive amounts of the acid will destroy the acetal moieties.

For release of a dialdehyde of General Formula (I), the diacetal (X) would be reacted with an acid such as hydrochloric acid to yield the free dialdehyde. Sodium hydroxide may be used to regenerate (X) from the salt form, if desired.

For a compound of Formula (IX), and thus of (X) and (I), where the two $ALK^1$ moieties are not the same, a single mole of the amine (V) or (VII) is reacted with the anhydride corresponding to the diacid chloride of formula (VIII), e.g. succinic anhydride. The thus-produced mixed carboxylic acid-amide is then reacted with a mole of a different amine of formula (V) or (VII) using a peptide coupling reagent such as (2-ethoxy-1-ethoxycarbonyl-1,2-diahydroquinoline, known as EEDQ, or 1,3-dicyclohexylcarbodiimide, known as DCC, to yield an amide of formula (IX) wherein the two $ALK^1$ moieties are not the same.

One skilled in the art will understand that concentrations of oxidized polyamines suitable for use herein are concentrations effective to induce the desired immunosuppressive response without exhibiting substantial cell toxicity. Effective concentrations may vary widely according to the particular cells it is desired to imnunosuppress, and the organism from which these cells are obtained. Exemplary concentrations for use herein may be extrapolated from the preferred concentrations for spermine dialdehyde These concentrations generally range from about 0.01 mM to about 0.2 mM, more preferably about 0.03 mM to about 0.1 mM, and most preferably about 0.03 mM to about 0.06 mM.

The immunosuppressive response induced by the techniques described herein, and the efficacy of the dosing, may also be measured in vitro by methods conventional to the art. This may be accomplished by the incubation of cells to be immunosuppressed with various concentrations of the compound, and comparison of the ensuing proliferation of these cell populations with that of control samples that have not been treated with the compound as described herein.

The oxidized polyamines are administered to the cells in any suitable physiologically compatible vehicle such as saline, phosphate buffer saline, methycellulose solutions, and the like. Solutions of the compound or homogeneous dispersions are preferred for such administration. The cells are allowed to incubate with the compound in a suitable growth medium such as minimum essential medium (MEM), RPMI 1640 and other suitable tissue culture media, to maintain cellular viability. This incubation generally takes place for a period of time sufficient to induce suppression of cell proliferation of at least about 25%, and preferably at least about 50%, as compared to control values of cellular growth. The incubation generally ranges for a period of time of about 10 minutes to about 1 hour, preferably about 10 minutes to about 30 minutes.

Representative of the therapeutic use of cells treated with oxidized polyamines, is the treatment of bone marrow extracts ex vivo with spermine dialdehyde prior to transplantation of these extracts into a patient. Treatment with the spermine dialdehyde can inactivate T lymphocytes without exhibiting toxic effects on the marrow cells themselves. This phenomenum has been shown to alleviate typical graft vs. host reactions, which are noted as being lethal to patients undergoing bone marrow transplantations. Suitable treatment parameters for such bone marrow extracts in human subjects may be the ex vivo treatment of cells as described above.

The present inventors have also discovered that direct in vivo administration of the compounds may also be effective to elicit an immunosuppressive response in an organism. For example, it has been discovered that in vivo administration of the compound spermine dialdehyde can greatly reduce graft vs. host reactions in a living organism. It has further been discovered that administration of this compound will

suppress the generation of cytotoxic T cells. It is believed that cytotoxic T cells play a role in the mediation of organ graft rejections, and thus, in vivo suppression of these cells will lead to prolonged graft survival. Hence, it should be appreciated that the in vivo administration of these compounds has far-reaching therapeutic advantages.

Skin graft prolongation has been used by immunologists to predict the efficacy of compounds in suppressing organ rejection. For instance, antiThyl antibody (mouse equivalent of Orthoclone OKT3) (R.M. Gorczyzynski, M. Boulanger and C. Lau. J. Immunol. 138:3197-3202 (1987)), FN18 (Frans J.M. Nooij and Margreet Jonker, "The effect on skin allograft survival of a monoclonal antibody specific for a polymorphic CD3-like cell surface molecule in rhesus monkeys". Eur. J. Immunol. 1987. 17:1089-1093), (monkey equivalent of Orthoclone OKT3) were all effective in suppressing skin graft rejection and Orthoclone OKT3 turned out to be effective in suppressing solid organ rejection in humans (Cosimi, A.B., Burton, R.T., Colvin, R.B., Goldstein, G., Delmonico, F.L., Laguaglia, M.P. Tolkoff-Rubin, N., Rubin, R.H., Herrin, J.T., and Russell, P.S., Transplantation 1981. 32:535). CsA, a potent immunosuppressive molecule for organ transplantation is also effective in skin graft experiments. Taken together, these data suggest that spermine dialdehyde, which is more effective than a comparable dose of CsA, should be at least as active as CsA in stopping organ rejection after transplantation.

Hypersensitivity is involved with allergic reactions (DeWeck, A. 1983. Regulation of IgE responses, New Trends in Allergy, J. Ring and G. Burg (eds.). Springer-Verlag, Berlin) and autoimmunity (allergic encephalomyelitis) (Weigle, W.O. 1980, Analysis of autoimmunity through experimental models of thyroiditis and allergic encephalomyelitis, Adv. Immunol. 30, 159). The fact that spermine dialdehyde suppressed DTH suggests that it might be useful in certain forms of autoimmunity. One injection in the footpad would suppress swelling and inflammation, suggesting that it can be used for treatment of inflamed joints in Rhematoid Arthritis (RA) patients through direct injection into the point. For RA patients experiencing acute flare of the disease, direct injection into the joint with prednisone is a very common treatment. Spermine dialdehyde could supplement or substitute for prednisone in the treatment of inflamed joints in RA patients.

Compounds administered therapeutically according to the method of the invention may be prepared as described above, and are also preferably dialdehydes synthesized from a diacetal. Formulations containing the oxidized polyamines or pharmaceutically acceptable salts thereof may also be used in this method, including ingredients such as conventional pharmaceutically acceptable carriers, like saline or sterile water, or ingredients to aid solubility or preservation of the formulation.

The preferred in vivo mode of administration of the compounds according to the method of this invention to achieve the desired immunosuppression is parenteral, more preferably intravenous or subcutaneous, and most preferably subcutaneous. It is not believed, however, that the specific mode of administration is critical to the practice of the present method so long as an effective amount of the compound enters the blood stream.

In certain embodiments, the present inventors have found that about 5-10 mg/kg of spermine dialdehyde administered intraveneously or subcutaneously was effective to suppress graft vs. host reactions, as detailed in the Examples section. However, it is believed to be well within the skill of a practitioner in the field to determine other appropriate doses of compounds and frequencies of administration to achieve the desired immunosuppressive response. Subcutaneous dosing of about 10 mg/kg i.p. - 20 mg/kg i.p. correlated well with the i.v. dosing. It is contemplated that for subcutaneous administration, those skilled in the art would recognize the necessity to increase the dose. Accordingly, the only practical limits are dictated by optimum efficacy, and hence, all such doses, frequencies of administration, and modes of administration are intended to be included within the subject method.

Efficacy of the method of the invention may be determined by observations of the clinical manifestations of graft vs. host reactions (either in the human or in one or more animal species) such as hunched back, diarrhea, alopecia, poor physical condition, wasting, and in the most drastic cases, death. Reduction of these symptoms would be an indication that the compound is exerting its effect, at the chosen dosing. Signs of toxicity for example, leukopenia, anemia, lack of reconstitution, and the like would indicate that the dosage should be reduced.

Indirect measurement of efficacy may also be useful in establishing and monitoring dosage levels. For example, this may be determined by assaying the cytotoxic T cell populations by mixed lymphocyte reaction assays, using peripheral T cells.

It has been reported in the literature that inhibitors of aldehyde dehydrogenase (ALDH) potentiate the cytotoxic action of cyclophosphamide on pluripotent hemopoietic stem cells and myeloid progenitor cells. Hilton, J. (1984) Cancer Res. 44, 5156-5160; Kohn, F.R., and Sladek, N.E. (1985) Biochem. Pharmacol. 34, 3465-3471; Kohn, F.R., Landkamer, G.L., Manthey, C.L., Ramsay, N.K.C., and Sladek, N.E. (1987) Cancer Res. 47, 3180-3185; Sahovic, E.A., Colvin, M., Hilton, J., and Ogawa, M. (1988) Cancer Res. 48, 1223-1226.

Studies were undertaken to determine if ALDH could exert similiar modulation to confer differential sensitivity to different cell types for SDA, particularly in purging bone marrow cells contaminated with T lymphocytes and/or tumor cells. Investigations indicate that the antineoplastic and immunosuppressive activity of SDA could be modulated, at least in part, by intracellular ALDH present in target cells. The involvement of ALDH was obvious with the following observations: 1) NAD linked cytosolic ALDH could oxidize SDA to a presumably nontoxic product (spermic acid); 2) murine leukemic cells L1210/CPA and L1210/0, having different levels of ALDH differ in their sensitivity to SDA and 3) the ALDH inhibitor, DEAB, potentiated the antitumor and lethal effect of SDA on L1210/CPA cells and on specific colonies of hemopoietic progenitor cells.

SDA could be used as a high affinity substrate for cytosolic ALDH in both liver and tumor cells. The known inhibitors, disulfiram, DEAB, and acrolein inhibited ALDH with SDA as substrate, consistent with previous reports on ALDH using other aldehydes. The potent inhibition by acrolein may be particularly important in toxicities associated with SDA treatment. Based on the chemical dynamics of the SDA molecule in solution and its comparison with the metabolism of cyclophosphamide, it can be postulated that SDA could give rise to acrolein by the process of $\beta$-elimination under favourable, base catalytic conditions. If acrolein produced by metabolism of SDA were to inhibit ALDH, there would be an increase in the formation of acrolein and an increase in the chances of unrelated toxic effects.

The increased expression of ALDH activity has been shown to be the mechanism of antitumor drug resistance to cyclophosphamide in two experimental murine leukemia cell lines (see above-cited references). ALDH activity was 200 fold higher in L1210/CPA cells compared to wild type sensitive L1210/0 cells. When these cells were treated with SDA, proliferation assays showed that L1210/0 cells were only 2 fold more sensitive than L1210/CPA cells. However, it does not necessarily undermine the impact of ALDH on SDA activity, because in certain studies, the present inventors have found that DEAB pretreatment resulted in equisensitivity for both cell types. Different isozymes of soluble ALDH have been reported to be present in various bone marrow derived cell lines and SDA may not interact with the isozyme(s) present in L1210/CPA as efficiently as cyclophosphamide derivatives. (Russo, J.E., and Hilton, J. (1988) Cancer Res. 48, 2963-2968).

Murine bone marrow cell proliferation, based on $[^3H]$-thymidine incorporation, indicated relatively less cytotoxicity at lower concentrations of SDA; DEAB pretreatment, however, caused dramatic enhancement of SDA action. It may be pointed out that the influence of ALDH on SDA activity would be relevant only at lower concentrations (< 20 $\mu$M). At higher concentrations of SDA, the nonspecific toxicity of the drug appears to predominate, irrespective of intracellular ALDH levels. A good correlation was observed between proliferation and appearance of hemopoietic progenitor colonies with respect to SDA dosage. In agreement with previous reports on cyclophosphamide, BFU-E and CFU-GM were found to be more sensitive to SDA in the presence of an ALDH inhibitor. Although the present inventor does not wish to be bound by theory, it is noted that single splenic T cells did not show a differential reponse to SDA in the presence of similar inhibitors, the selective purging effect of SDA on T cells observed earlier may be attributed to the different ADH levels in different cell types.

Bone marrow toxicity and tumor cell or immune responsive T lymphocyte resistance are the two major limiting factors in bone marrow transplantation. Identification of different ALDH isozymes (in hemopoietic cells and contaminating cells) using specific immunological probes and inhibitors would facilitate the development of optimal conditions to selectively sensitize the uneconomic species to SDA and thereby possibly offer an enhanced therapeutic margin of safety.

The following examples more specifically describe certain embodiments of the present invention but should not be considered limitative thereof.

## EXAMPLES

I. Chemical Synthesis of N′N′-Bis(3,3-diethoxypropyl)-1,4 butanediamine nitrate spermine (bis-acetal)

All materials were obtained from Aldrich. All reactions are run under an atmosphere of nitrogen, and solvents are either HPLC grade (methanol, methylene chloride, NMP, ethyl acetate) or anhydrous "Gold Label" grade (THF).

1. Preparation of N-(3,3-diethoxypropyl)phthalimide (Compound 1), A mixture of 3-chloropropionaldehyde diethyl acetal (16.7 g, 0.10 mol) and potassium phthalimide (18.5 g, 0.10 mol) in N-methylpyrrolidinone (200 ml) was stirred overnight at 125 °C under nitrogen. The resulting solution was cooled and poured into water (400 ml) and the resulting mixture was extracted with ether (2 x 200 ml). The ether extracts were washed with water (2 x 100 ml), dried ($MgSO_4$), filtered, and evaporated to leave an orange oil.

Chromatography on silica (200 g) with 95:5 methylene chloride-ether provided pure Compound 1 as a pale orange oil (16.5 g, 60%).

2. Preparation of 3,3-diethoxypropylamine (Compound 2), A solution of N-(3,3-diethoxypropyl)phthalimide (1) (16.5 g, 59.5 mmol) and hydrazine (3.8 g, 120 mmol) in methanol (300 ml) was stirred at reflux for 4 hours (a mechanical stirrer was necessary as the mixture thickened considerably). The mixture was cooled with continued stirring, and filtered, washing the precipitate with methanol. The filtrate was evaporated to a semi-solid mass, which was taken up in methylene chloride (150 ml) and filtered again. The filtrate was concentrated and the residue was distilled in vacuo to provide Compound 2 as a colorless liquid (6.5 g, 70%). (The reported boiling point is ca. 60 °C at 4 mm of Hg, ca. 70 °C at 20 mm of Hg.).

3. Preparation of N,N′-bis(3,3-diethoxypropyl)succinamide (Compound 3), A solution of succinyl chloride (3.25 g, 21.0 mmol) in methylene chloride (50 ml) is added dropwise with vigorous stirring to a cold (-50 °C) solution of Compound 2 (6.14 g, 41.7 mmol) and triethylamine (6.3 ml, 45 mmol) in methylene chloride (200 ml). The mixture was allowed to warm to room temperature and stirred under nitrogen overnight. The solvent was evaporated and the residue was taken up in ether (200 ml). The resulting suspension was filtered, and the filtrate evaporated. The residue was chromatographed on silica (150 g) with 6:1 ethyl acetate-isopropanol, and the product recrystallized from hexane to provide 3 as a colorless solid, mp 89-90 °C (4.2 g, 53%). The material decomposed over a period of two months when stored at room temperature.

4. Preparation of N,N′-bis(3,3-diethoxypropyl)butane-1, 4-diamine dinitrate (Compound 4), A mixture of Compound 3 (4.0 g, 10.6 mnol) and lithium aluminum hydride (0.84 g, 22 mmol) in tetrahydrofuran (200 ml) was refluxed under nitrogen for 40 hours, then cooled. Water (1.0 ml), 15% aqueous sodium hydroxide (1.0 ml), and more water (3 ml) were added dropwise with vigorous stirring. The mixture was stirred for 30 minutes, then filtered. The filtrate was evaporated, leaving a pink oil. This was taken up in ethanol (20 ml), diluted with 50 ml ether, and concentrated nitric acid (1.3 ml) was added dropwise with vigorous stirring. A precipitate formed quickly, which was collected by filtration and washed with ether. Drying in vacuo provided Compound 4 as a white solid, mp 145-146 ° (2.5 g, 50%).

B. Preparation of Spermine Dialdehyde from Spermine bis-acetal

Spermine bis-acetal is dissolved in 1N HCl at a concentration of 100 mM (47.5 mg/ml). The solution is incubated in a 37 °C water bath for an hour. At the end of the incubation period, the 100 mM solution is diluted to 20 mM with purified water. The pH of the 20 mM solution is adjusted to between 5.0 and 6.5 using 10N NaOH. This pH adjusted 20 mM solution is the stock for all test material.

Preparation of Control Article

Control vehicle stock solution is obtained by titrating a 0.2M HCl solution with 10N NaOH and adjusting the pH to between 5.0 and 6.5. To obtain a control working solution, the stock solution is then diluted accordingly as with the test solution.

Material Used for Bioassays

The following materials were used in the Examples. Purified phytohemagglutinin (PHA-P) was obtained from Wellcome. Pokeweed mitogen (PWM) was obtained from Gibco. Tritated thymidine and chromium (51Cr) were obtained from New England Nuclear. Con A was obtained from Sigma. C57/B6, DBA/2, C3H and Balb/C mice were obtained from the Jackson Laboratory. Growth medium for human cell lines consisted of ð-minimum essential medium (ð-MEM) (Gibco) supplemented with penicillin (Gibco 50 Mg/ml), streptomycin (Gibco, 100 Mg/ml), L-glutamine (Gibco, 2.0 mM) and 2-10% fetal calf serum (FCS, Gibco). The medium for the T cell proliferation assay was the same except RPMI 1640 (Gibco) was used instead of ð-MEM; 10% FCS was used at all times.

C. Preparation of Mouse Bone Marrow and Spleen Cells

Mice were sacrificed by cervical dislocation and disinfected by dipping in a Dettol solution. Bone marrow cells were obtained from suitable long bones by flushing with a 0.25 gauge needle. Cells were then washed three times with RPMI before use.

Spleen cells were prepared by passing the spleen through a fine wire mesh. Cells were resuspended in RPMI and washed three times before use.

D. Preparation of Human B and T Lymphocytes and Lymphocyte Proliferation

Peripheral blood lymphocytes (PBL) were obtained from the blood of normal donors by using standard Ficoll-Hypaque gradient techniques. The T and non-T cells were separated by standard sheep red blood cell (SRBC) rosetting technique. Briefly, 5 x $10^6$/ml PBL were incubated with 1% neuraminidase-treated SRBC, and rosetted cells were separated from non-rosetted cells in a Ficoll-Hypaque gradient. The rosetted cells were designated as T cells. Purified B lymphocytes were obtained from non-rosetted cells by removal of residual T cells using the Pan T monoclonal antibody OKT 11 and

complement.

E. Human Lymphocyte Proliferation

T cell proliferation: 7 x $10^4$ - $10^5$ human T lymphocytes were cultured in flat bottom microtiter plates (Flow) in the presence of 0.1% PHA-P or 1/64 PWM for 4 days. 3H-thymidine (0.1 MCi/well) was added for the last 6 hours of the culture period. T cell dependent B cell proliferation: 4 x $10^4$ T cells and $10^5$ B cells were cultured together in the presence of 1/64 dilution of PWM for 4 days. 3H-thymidine was added for the least 6 hours of the culture period. B cell proliferation was carried out in exactly the same manner with no T cells added to the culture. In all cases, cells were subsequently harvested onto filter paper using a multiple-channel automated cell harvester (Flow) and washed repeatedly with distilled water. Cell associated radioactivity was determined by scintillation counting in an automated counter. All of the results were expressed as % response calculated according to the formula:

$$\% \text{ response} = \frac{\text{cpm of supernatant-treated cultures}}{\text{cpm of control cultures}} \times 100$$

F. Mouse Lymphocyte Proliferation

Mouse spleen cells were cultured for 3 days in flat bottom microtiter relates at $10^5$ cells per well in the presence of 2 mg/ml of Con A. 3H-thymidine (0.1 microcurie/well) was added for the last 4-6 hours of the culture period. Cells were harvested as described above.

G. Bone Marrow Transplantation in the Mouse Model

In bone marrow transplantation, bone marrow cells from normal donor mice were transplanted to totally histoincompatible and lethally irradiated recipients. All recipient mice received 9.5 Gy (950 rads) total body irradiation on day 0 and were then housed in microisolator cages thereafter. They were given autoclaved rodent laboratory chow throughout the whole study and also were allowed to feed ad libitum. Recipients also received 1.5 mg/kg of gentamicin subcutaneously for 20 days after irradiation. On the day of irradiation, bone marrow cells mixed in 3:1 ratio with spleen cells were removed from donors and incubated for an hour with different concentrations of spermine dialdehyde for 1 hour. Spleen cells were added to intensify subsequent graft vs. host disease (GVHD). After incubation, cells were washed 3 times and then injected intravenously into irradiated recipients. Signs of GVHD and survival were scored every day thereafter.

H. Graft Vs. Host Reaction in Rat (Popliteal Lymph Node Study) According to Method of Ford et al. Transplantation, Vol. 10, pp. 258-266 (1970).

Graft vs. host reaction in rats was measured by popliteal lymph node enlargement induced by reinjection of parental spleen cells into F1 recipients. (Lewis x BN) F1 rats were divided into groups of 10 and given daily IV injections of different doses of the test compound (spermine dialdehyde) or control preparation. Two days after the first injection, they received 9 x $10^6$ spleen cells from a parental Lewis strain (0.3 ml of a 27 x $10^6$ cell/ml preparation was injected under the skin of the ventral surface of each of the recipient's right hind foot). Injection of test compound and control article continued for 6 more days. On the seventh day, rats were sacrificed by $CO_2$ treatment. Both left and right politeal lymph nodes were removed and cleaned of any adhering tissue. The extent of enlargement was measured by weighing both left and right lymph nodes and differences between the two were calculated.

I. Generation of Cytotoxic T Cell in Mice (In Vivo)

In vivo generation of cytotoxic T cells was performed according to the method described by Faanes et al., Clin. Esp. Immunol., Vol. 27 pp. 502-506 (1977). C57BL mice were arranged into groups of 10 and injected IP with different doses of spermine dialdehyde or control preparation. Two days after first injection, they received i.p. injection of $10^7$ DBA spleen cells which were prepared as described previously. Ten days after cell injection, C57 mice were sacrificed by cervical dislocation and cytotoxic T cells generated in the spleen were quantified by measuring the ability of those cells to lyse 51Cr labelled PB15 target cells (a mastocytoma, cells of DBA origin or Con A stimulated DBA spleen blasts). P815 cells or blasts were suspended at $10^7$ cells/ml and incubated with 200 MCi of 51Cr for 1 hour at 37°C. Cells were then washed 3 times and then mixed with C57BL spleen cells at ratios of 20:1, 10:1 and 5:1 (C57BL:P815) in a V-bottom microlitre plate with a final volume of 200 ml. Cells were spun for 6 minutes and incubated for 4 hours at 37°C. After incubation, 100 Ml of supernatant was removed and counted in the gamma counter. The percentage of cytotoxic T cells was calculated:

$$\% \text{ total cytotoxicity} = \frac{\text{51Cr released by spleen cells - spontaneous release}}{\text{Total release - spontaneous release}} \times 100$$

J. Acute Toxicity

Two pairs of Swiss Webster mice were injected intraperitoneally at 200 mg and 400 mg/kg respectively and observed for toxic effects. Control groups were injected with control preparation. Two weeks after dosing, animals were necropsied and organs were examined for abnormalities.

K. Aldehyde Test

Aldehyde test was performed according to the method described by Sawicki et al., Analytical Chem., 33, 93-96 (1961). Briefly, 50 Ml of 0.4% solution of 3-methyl-2-benzothiazolone hydrazone (MBTH) was added to 50 Ml of the test solution. The mixture was allowed to stand at room temperature for 30 minutes, then 200 Ml of a 0.2% solution of ferric chloride was added and the mixture was then left at room temperature for 10 minutes. Six hundred and fifty Ml of acetone was then added to the sample with slow agitation and the color intensity of the sample was quantitated by spectrophotometric reading at wavelength 670.

L. In a skin graft transplant experiment, Balb/c ($H2^d$) skin grafts were transplanted onto C3H mice according to the procedure described in 1. Spermine dialdehyde was administered s.c, right after transplantation and continued throughout the study. Cyclosporin A (CsA) was run along as a positive control.

M. Delayed Type Hypersensitivity

Delayed type hypersensitivity was induced in the footpad of B6D2F1 mice by using sheep red blood cells (SRBC) as the antigen. Briefly, 0.2 ml of 0.01% SRBC was injected i.v. into B6D2F1 mice. Four days later, they were challenged in the footpad with 50 µl of 20% SRBC. Swelling of the footpad was measured 24, 48, and 72 hours later. Spermine dialdehyde was administered i.p. initiating from the day before immunization. After challenge in the footpad, B6D2F1 mice were given an intramuscular (i.m.) injection beside the challenge site one hour after challenge. Some mice received no i.p. administration and were only given one i.m. injection after the challenge. Results are shown in Figure 7.

N. Bioassay For Screening of Analogues

In order to establish suitable bioassays for screening of analogues, it was considered appropriate to ascertain whether or not SDA is equal to or better than the well-known polyamine interfering agents. As shown in the table below, the potency of SDA was similar to certain polyamine analogues, bis (ethyl) spermine (Porter et al. 1987) and to DFMO, an irreversible inhibitor of ornithine decarboxylase (Metcalf et al. 1978).

Moreover, unlike DFMO and bis-spermine, SDA was capable of inhibiting both of the key enzymes of polyamine biosynthesis, thereby eliminating the compensatory response and ensuring complete inhibition of polyamine synthesis. SDA, like the other two agents did not inhibit the enzymes of unrelated pathways indicating the specific interaction with polyamines. Similar bioassays may be used by those skilled in the art to identify desired SDA analogues taught herein.

| Drug | T cells Proliferation | Leukemic cell growth | Ornithine decarboxylase | AdoMet decarboxylase | Acid phosphatase | Alkaline phosphatase |
|---|---|---|---|---|---|---|
| Spermine Dialdehyde | +++ | +++ | +++ | +++ | - | - |
| * Difluoro Methyl Ornithine | ++ | ++ | ++++ | ± | - | - |
| ** Bis (ethyl) Spermine | +++ | +++ | ++++ | - | - | - |

* Metcalf *et al.*, J. Am. Chem. Soc. 100, 2551-2553 (1978),

** Porter *et al.*, Cancer Research 47, 2821, 1987

II. RESULTS

A. Effect of Spermine Dialdehyde on Human T and B Lymphocytes

Human lymphocytes were prepared and separated into T and B lymphocytes as described previously. T and B lymphocytes were incubated with different concentrates of spermine dialdehyde for

1 hour at 37° C. Cells were washed 3 times with RPMI and were then set up in a PHA driven T cell proliferation assay or pokeweed mitogen (PWM) driven B cell proliferation assay. Results are illustrated in Figure 1. As can be seen, spermine dialdehyde inhibited T cell proliferation by 85% at 0.62 x $10^{-2}$ mM whereas a much greater quantity was required to suppress B lymphocyte proliferation. Thus, spermine dialdehyde preferentially suppresses T cell proliferation with a much less suppressive effect of B lymphocytes. Furthermore, these data suggest that spermine dialdehyde could irreversibly inactivate T lymphocytes after a brief incubation of 1 hour (5-10 minute incubation was in fact sufficient - data not shown). This novel property distinguishes spermine dialdehyde from other immunosuppressive molecules such as cyclosporine A or prednisone which are all reversible in nature.

B. Effect of Spermine Dialdehyde on Mouse Spleen and Bone Marrow Cells

Mouse bone marrow and spleen cells were each incubated for 1 hour with different concentrations of spermine dialdehyde. Cells were then washed 3 times with RPMI and set up in a Con A driven proliferation assay. Results are depicted in Figure 2. Spleen cells, as can be seen, were much more susceptible to suppression mediated by spermine dialdehyde than bone marrow cells.

C. Ex Vivo Treatment of Bone Marrow Graft With Spermine Dialdehyde Alleviated Graft Vs. Host Disease in Recipients and prolonged Survival Time

Bone marrow and spleen cell mixtures (3:1 ratio) from C57BL mice were incubated with control preparation or various concentrations of spermine dialdehyde for 1 hour Cells were then washed extensively and injected intravenously into lethally irradiated histoincompatible AKR mice. Signs of graft vs. host diseases such as hunched back, diarrhea, alopecia, and physical conditions were recorded daily. Survival times of reconstituted mice are shown in Figure 3. Control mice showed severe signs of acute GVHD initiating around 10 days after transplantation. Mice receiving bone marrow treated with spermine dialdehyde manifested only mild signs of GVHD. The very high dose groups of 0.2mM exhibited marrow toxicity and animals died around the same time as the irradiated controls probably due to lack of reconstitution. Mice receiving marrow treated with lower doses of spermine dialdehyde (0.03-0.06) showed significantly prolonged survival time. Thus treatment with lower doses of spermine dialdehyde inactivated T lymphocytes without being toxic to marrow celis and allowed animals to reconstitute with little sign of GVHD. These data suggest that spermine dialdehyde couid be employed to treat human bone marrow samples ex vivo before bone marrow transplantation to alleviate subsequent GVHD which is usually lethal to the patients.

D. Suppression of Graft Vs. Host Reaction in Rats by In Vivo Injection of Spermine Dialdehyde

Graft vs. host reaction was generated in the form of popliteal lymph node in F1 recipients by subcutaneous injection of parental spleen cells. Spermine aldehyde was administered intravenously starting 2 days before parental spleen cell injection and continued until the day of lymph node measurement. Increase in popliteal lymph node swelling was scored by difference in weight between test (left) and control (right) lymph node. Results were shown in Figure 4. Substantial swelling was observed in test lymph nodes. Intravenous injection of spermine aldehyde significantly suppressed the GVH reaction as depicted by diminutive popliteal lymph node swelling. This GVH reaction is a typical manifestation of MHC class II reaction involving allogenic T lymphocytes. Spermine dialdehyde, when given intravenously suppressed this reaction, suggesting that this molecule mediates the same effect in vivo as in vitro, that is, it acts as a potent suppressor of T cell reactivity.

E. In Vivo Suppression of Generation of Mouse Cytotoxic T Cells by Spermine Dialdehyde

About 10 days after receiving DBA spleen cells, C57BL mice will generate cytotoxic T cells in their spleens which lyse P815 mastocytoma cells bearing DBA specific antigens. Since cytotoxic T cells play an important role in the mediation of organ graft rejection, in vivo suppression of cytotoxic T cell generation may lead to prolonged graft survival. Spermine dialdehyde, when administered i.p., suppressed the generation of cytotoxic T cells in a dose related manner (Figure 5). Dose of 10 mg/kg i.p. appeared to attain maximum suppression which corresponds well with the dose required to achieve maximum suppression of GVH reaction in vivo (5 mg/kg i.v., Figure 4).

The two models described above demonstrated for the first time that synthetic spermine aldehyde is effective as an immunosuppressive compound in vivo.

F. Acute Toxicology Results

Two pairs of Swiss Webster mice were injected intraperitoneally at 200 mg and 400 mg/kg for toxic effects. Spermine dialdehyde appeared to be acutely irritating in these test animals as indicated by squinting and writhing. Mice receiving 200 mg/kg exhibited no other toxic effect whereas those injected with 400 mg/kg showed abdominal or whole body edema for five days. At necropsy 14 days after injection only the 400 mg/kg group showed any positive findings which consisted of a mildly distended abdomen and scab formation at the injection site. This data differed significantly from results reported by

Israel et al Supra which showed that the LD100 of this molecule is 40 mg/kg.

G. Prolongation of Skin Graft Survival

As depicted in Figure 6, CsA and all 3 doses of spermine dialdehyde prolonged skin graft survival, especially 10 mg/kg and 20 mg/kg of spermine dialdehyde.

B6D2F1 mice receiving both i.p. administrations and one injection in the footpad showed significant reduction in swelling when measured 24 hours after challenge and also 48 hours later. Mice receiving 1 injection in the footpad did not exhibit reduction in swelling 24 hours later, but did so 48 hours later.

Results suggest that mice receiving spermine dialdehyde both systemically and locally showed reduced swelling measured at 24 or 48 hours after challenge. Mice receiving spermine dialdehyde only after challenge showed swelling similar to that of mice receiving control vehicles 24 hours later. However, 48 hours later, reduced swelling compared to that of the control group was observed.

Delayed type hypersensitivity is a T-cell mediated immune response. After immunization with the antigen, mice would be sensitized with specific T cells responding to the antigen. These T cells will migrate to various parts of the body including the footpad. During challenge with the same antigen, these T cells will be reactivated and result in the release of lymphokines, some of which will be chemotactic for monocytes, macrophages and neutrophils. Accumulation of these cells then leads to a local inflammatory response causing substantial swelling. When spermine dialdehyde was administered i.p. before and right after sensitization, it probably suppressed T cell responses to the antigen and after challenge the injection at the footpad suppressed T cells and other cells involved in inflammation (reduction of swelling was not significant if the footpad injection was omitted). However, if injection was given only after challenge in the footpad, suppression of inflammatory response could also be observed 48 hours later. These findings suggest that spermine dialdehyde suppressed T cell response to the antigen both at the sensitization and inflammatory stages. Suppression, however, was most significant when spermine dialdehyde was administered before and during sensitization and also after challenge. Combined injections suppressed both the generation of sensitized T cells and the de novo inflammatory response.

H. Results of a Bioassay of SDA Analogues - Comparative Studies

Comparative SDA analogues 1-6, listed in the Table below, represent substitution of aldehyde end groups by ester or ketone and reduction in carbon chain length beside the nearest nitrogen. Both methyl and ethyl esters were ineffective in inhibiting the proliferation of T cells/tumor cells or polyamine synthesis.

Both methyl ketone (3) and its chloroderivative (4) might show more chemical and metabolical stability than SDA, and one could postulate that they might be suitable for systemic use. However, preliminary in vitro alkylation studies revealed these compounds to be nonspecific alkylating agents, presumably causing DNA breaks/cross-linking, to result in cell death. Subsequently, the chloride was removed and C-chain was reduced, while retaining the ketone group to yield compounds 5 and 6. The reduction in C-chain from propyl to methyl or ethyl resulted in complete loss of activity (see Table).

Since the aldehyde group in SDA appeared to be critical to antiproliferation activity with intervention in polyamine metabolism, the present inventors have initially sought an aldehyde analogue with a longer C-chain beside the central nitrogen (7). The butyl configuration should make the aldehyde relatively resistant to β-elimination reactions, yielding a more stable in vivo agent than SDA. Butyl dialdehyde (7) was found to be less potent than SDA in proliferation assays (Fig. 8). However, the compound selectively inhibited adenosylmethionine decarboxylase (Fig. 9), indicating specific interaction with polyamine biosynthesis. The inhibition curves for SDA and butyl dialdehyde were superimposable with $IC_{50}$ values of 0.12 - 0.13 x $10^3$M.

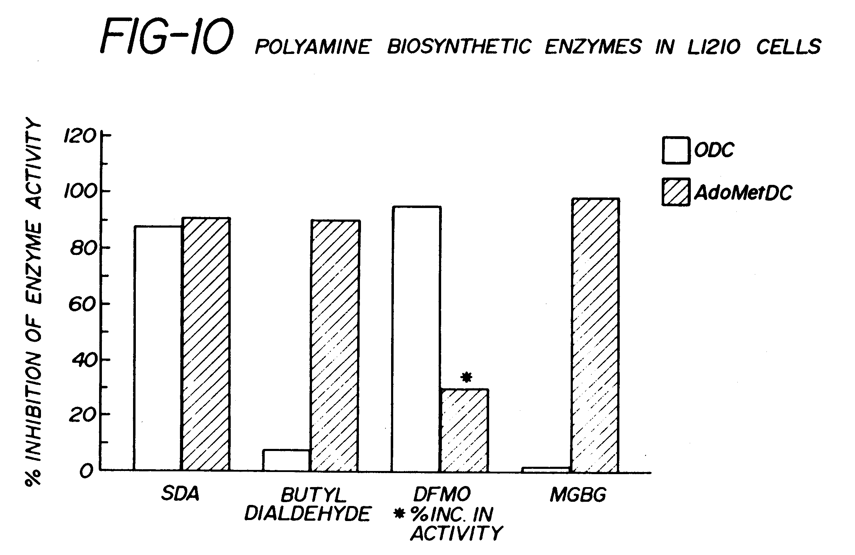

Since DFMO caused a compensatory increase in AdoMetDC, and methyl-glyoxal-(bisguanylhydrazone) (MGBG) inhibited AdoMetDC (Fig. 10), the present inventors have discovered that a SDA-like compound is ideal for the inhibition of polyamine biosynthesis. Analogues which inhibit both the key enzymes and are metabolically more stable than SDA, such as butyl dialdehyde, and its analogues, might be very useful in this regard. Moreover, in vivo characterization of butyl dialdehyde may also offer further insights into the mechanism of action of SDA.

In summary, both ketone and aldehyde analogues of SDA have been shown to be suitable for inhibition of the growth of T cells and tumor cells. However, the studies to date have indicated that only aldehydes cause a suppression of polyamine biosynthesis, and accordingly, are preferred as claimed herein. Chloromethyl ketone was a highly alkylating and nonspecific toxic agent. Analogues with shorter carbon chain beside the ketone group showed some attenuation of the biological activity. Based on these observations, aldehyde analogues with an increased C-chain in the side or central part of the molecule, or with an asymmetric configuration (eg. a monoaldehyde) may also be used in the methods described herein.

COMPARATIVE STUDIES

SDA AND ITS ANALOGUES STRUCTURE - FUNCTION RELATIONSHIP

| | T cells Proliferation | Leukemic cell growth | Ornithine decarboxylase | AdoMet decarboxylase |
|---|---|---|---|---|
| 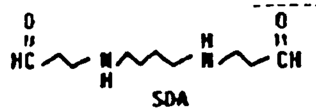 SDA | +++ | +++ | +++ | +++ |
| ANALOGUE # 1 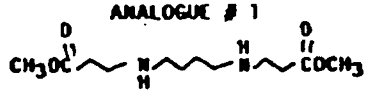 | - | - | - | - |
| ANALOGUE # 2 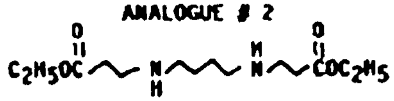 | - | - | - | - |
| ANALOGUE # 3 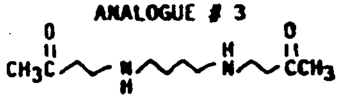 | ++ | + | - | - |
| ANALOGUE # 4 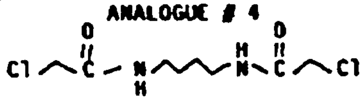 | +++ | +++ | - | - |
| ANALOGUE # 5 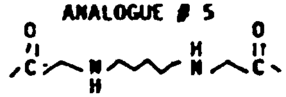 | - | - | - | - |
| ANALOGUE # 6 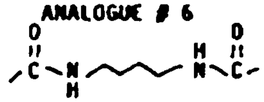 | - | - | - | - |
| ANALOGUE # 7 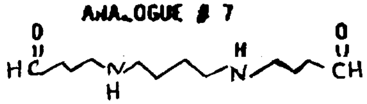 | ++ | ++ | - | +++ |

I. Studies on the Effect of Aldehyde Dehydrogenase in Relation to SDA Treatment

Materials and Methods

Materials: SDA was prepared by acid hydrolysis of the corresponding bioacetal supplied by the Department of pharmaceutical Development at our Institute. [$^3$H]-Thymidine was purchased from New England Nuclear, Boston, MA. Conconavalin A, horse serum, RPMI-1640 and HEPES were from Gibco, Grand Island, NY. NAD and NADP were purchased from Boehringer, Mannheim. Acrolein and disulfiram were obtained from Aldrich Chemical Co., Milwaukee, WI. propionaldehyde and benzaldehyde were from Eastman Kodak Chemical Co., Rochester, NY. Diethyl aminobenzaldehyde (DEAB) was kindly provided by Dr. John Hilton, Johns Hopkins School of Medicine, Baltimore, MD.

Cell Culture: Murine lymphocytic leukemia cell lines, L1210/CPA and L1210/0 (also known as Le/CPA and Le/0) were kindly provided by Dr. F. Struck, Southern Research Institute, Birmingham, AL. Cells were grown in RPMI-1640, supplemented with 10% horse serum, 100 units/ml penicillin and 100 $\mu$g/ml streptomycin. The cultures were incubated at 37°C in a humidified atmosphere with 5% $CO_2$ in air. Bone marrow cells were flushed from femurs of mice with RPMI-1640, using a tuberculin syringe fitted with a 25 gauge meedle. For spleen cells, spleens were removed and passed through a fine wire mesh to get a single homogenous cell suspension. In either case, cells were centrifuged, washed twice and resuspended in the drug exposure medium (see below). Cell viability was determined by trypan blue exclusion and the cell suspension was diluted to the desired concentration.

Murine leukemic cells and bone marrow/spleen cells were treated with SDA (1 x $10^{-6}$ M to 5 x $10^{-5}$ M) in RPMI-1640 or PBS based solution. Wherever mentioned, cells were pretreated with DEAB (10-30 $\mu$M) for 30-60 min at 37°C and then exposed to SDA.

Cell Proliferation Assay: For [$^3$H]-thymidine incorporation, drug or vehicle treated bone marrow or spleen cells were plated in RPMI-1640, containing 10% fetal bovine serum, 10 $\mu$M $\beta$-mercaptoethanol, 25 mM HEPES and 2 mM glutamine. Con-A (4 $\mu$g/ml and 8 $\beta$g/ml) was used as mitogens for bone marrow and spleen cells. Three days later, [$^3$H]-thymidine (0.2 $\mu$Ci/well) was added and cells were harvested after 12-18 hrs. L1210/CPA and L1210/0 cells (0.1 - 1 x $10^5$ cells/well were plated in RPMI-1640 containing 10% horse serum. [$^3$H]-thymidine was added after 24 hrs and cells were filtered on GF/B filter paper for scintillation counting.

ALDH Assay: Cultured murine leukemic cells and livers (excised from mice immediately after cervical dislocation) were homogenized in 3 volumes of ice-cold 1.15% KCl and 1 mM EDTA. The homogenate was centrifuged at 100,000 g for 45 min and the resulting supernatant was used to measure cytosolic ALDH activity. For particulate ALDH assay, the pellet (and homogenate for total ALDH) was resuspended in homogenizing buffer and treated with 0.25% Triton® X-100 for 30 min at 4°C. The solubilized preparations were centrifuged at 48,000 g for 30 min and the supernatants were drawn off for ALDH determinations.

ALDH activity was measured by monitoring the reduction of NAD or NADP for 5-6 min at 340 nM at 37°C in DU70 spectrophotometer (Beckman). The assay mixture contained 50 mM potassium phosphate buffer, pH 7.4, 2.0 mM NAD (or NADP) and 1.0 mM EDTA. The reaction was started by adding the substrate (SDA or other aldehyde) to the prewarmed (5 min at 37°C) assay mixture containing enzyme preparation. Pyrazole (0.1 mM) was added particularly for liver enzyme, to inhibit alcohol dehydrogenase. The apparent $K_m$ and maximum velocity (Vmax) values were calculated using a Lineweaver-Burk plot based on the estimation of the slope by regression analysis. Enzyme activity was expressed as mU (nmoles/min) per mg of protein. Protein contents were determined by the Bradford method, using bovine serum albumin as the standard.

Hemopoietic Colony Formation Assay: Hemopoietic colony forming cells were cultured as described in the art by Iscove & Sieber with minor modifications. SDA/DEAB treated or vehicle treated cells were added to murine hemopoietic culture mixture with erythropoietin and conditioned medium (Terry Fox Laboratories, Vancouver, Canada). The mixture was vortexed and 1-2 ml portions (1 x $10^5$ bone marrow cells) were plated in duplicate in 35 mm plastic petri dishes (lux suspension dishes, Flow Laboratories). The culture dishes were incubated in a humidified atmosphere (95% air : 5% $CO_2$) at 37°C. Colonies were counted on day 8 or 10 for BFU-E, CFU-GM and CFU-mix. Colony types were confirmed with Wright/Giemsa Stain.

Results: Initial studies were designed to investigate the properties of ALDH with SDA as the substrate. In the cytosolic fraction of mouse liver and leukemic L1210/CPA cells, both propionaldehyde and SDA acted as high affinity substrates (see Table). The mean $K_m$ values of SDA for L1210/CPA and liver enzyme were 41.6 $\mu$M and 48.5 $\mu$M, respectively. The specific activity of ALDH (on per mg protein basis) was 1.5 times higher in the liver, as compared to L1210 cells (see Table). With propionaldehyde (1.0 mM) as the substrate for tumor cell and liver enzymes, the specific activity was much higher than the SDA-linked enzyme.

Therefore, the specific isozyme(s) interacting with SDA and propionaldehyde may not necessarily be the same or the higher affinity of propionaldehyde for cytosolic ALDH (see Table) may account for the increased enzyme expression.

Cyclophosphamide sensitive L1210/0 cells did not exhibit any detectable enzyme activity with SDA or other aldehydes. Similarly, ALDH activity could not be measured with SDA (up to 2.5 mM) in the mitochondrial fraction of L1210/CPA or mouse liver.

Known inhibitors of ALDH were used to confirm the identity of the enzyme involved in the oxidation of SDA (see Table). Disulfiram and DEAB inhibited ALDH activity with mean $IC_{50}$ values of 4.3 µM and 0.055 µM, respectively. Pyrazole, an inhibitor of alcohol dehydrogenase, had no effect on L1210/CPA cytosolic enzyme with SDA as the substrate (data not shown). Acrolein, a highly reactive $\alpha,\beta$ unsaturated aldehyde was also a potent inhibitor of cytosolic ALDH ($IC_{50}$-2.5 µM, (see Table). The activity of NADP-linked cytosolic ALDH, measured in L1210/CPA cells with SDA as substrate, was in the range of 3.0-3.5 mU/mg protein. The NADP-linked ALDH was found to be far less sensitive to acrolein than the NAD-linked enzyme (50% inhibition at 50-100 µM acrolein).

Both disulfiram and acrolein were very toxic to tumor cells and hemopoietic progenitor cells ex vivo at concentrations much lower than that needed for complete inhibition of ALDH. On the other hand, DEAB, besides being the most potent ALDH inhibitor in vivo and in vitro, showed negligible cytotoxicity even up to 50 µM. Therefore, in subsequent experiments, DEAB was chosen to study the survival of leukemic cells and myeloid progenitor cells after treatment with SDA.

L1210/CPA and L1210/0 cells treated with SDA showed a concentration dependent decrease in [$^3$H]-thymidine incorporation. L1210/0 cells appeared to be more sensitive to SDA as compared to L1210/CPA, with mean $IC_{50}$ values of SDA being 4.66 µM and 8.3 µM, respectively. Pretreatment of cells with DEAB (20 µM) potentiated the cytotoxicitty of SDA in L1210/CPA cells, while having little or no effect on L1210/0 cells. Cell viability estimated immediately after SDA treatment was the same as untreated cells (90%), except for higher concentrations (>25 µM) of SDA. However, the viability of SDA treated cells dropped sharply over a 12-24 hr period and it appeared as if most of the cells were "programmed to death" during SDA exposure. The remaining cells (< 25% viability after 2 days) continued to proliferate and after 6 days of SDA treatment, [$^3$H]-thymidine incorporation in these cells was similar to untreated cells, cultured for the same duration.

Murine bone marrow cells, treated with SDA in PBS based medium and cultured in RPMI-1640 - 10% FBS for 3 days, showed a dose dependent decrease in [$^3$H]-thymidine incorporation. DEAB (30 µM) pretreatment to these cells resulted in higher susceptibility at lower concentrations of SDA. Under our experimental conditions, ALDH activity could not be detected in homogenates of bone marrow cells. More sensitive conditions, such as specific antibody probes, are suggested to estimate the level of ALDH in these cells. SDA treatment (8-12 µM) abolished Con A-induced murine spleen cell proliferation, addition of DEAB (20 µM), however, had no significant effect on the potency of SDA.

Qualitatively similar observations on the role of ALDH were obtained with the bone marrow cell proliferation and the formation of myeloid colonies. SDA treatment caused almost total suppression of BFU-E and CFU-GM colonies at 50 µM concentration. However, at lower concentrations of SDA (10-25 µM), DEAB pretreatment further potentiated the sensitivity of myeloid colony forming cells after SDA exposure. Control colony formation ranged from 16-22 colonies and 170-210 colonies per 1 x $1o^5$ nucleated cells for BFU-E and CFU-GM, respectively.

Cytosolic ALDH in L11210/CPA Cells

| Substrate Inhibitor | Km(μM) | Specific (mU/mg Protein |
|---|---|---|
| SDA | 41.6 ± 5.1 | 6.8 ± 1.6 |
| | 48.5 ± 3.3* | 10.5 ± 3.2* |
| Propionaldehyde | 26.5 ± 4.9 | 10.5 ± 3.2 |
| | 14.0 ± 2.8* | 22.5 ± 3.9* |

Inhibition ($IC_{50}$, μM); SDA (1.0 mM) as substrate

| | |
|---|---|
| Disulfiram | 4.3 ± 1.1 |
| DEAB | 0.0555 ± 0.015 |
| Acrolein | 2.5 ± 0.63 |

ALDH activity was measured by monitoring the reduction of NAD at 340 nm, as described above. For inhibition studies, enzyme preparation was pretreated with inhibitor for 5 min at 37°C. Values are ± SEM from 3-4 separate experiments.

*Cytosolic ALDH in mouse liver.

## Claims

**1.** A method for inducing an immunosuppressive response in living cells <u>in</u> <u>vitro</u> which comprises administering to said cells, in substantially pure form and in an amount effective to induce said response, a compound of General Formula I:

$$OCH - ALK^1 - NR^2 - CH_2 - ALK^2 - CH_2 - NR^2 - Z$$

wherein

$ALK^1$ is independently alkylene;

$R^2$ is independently hydrogen or $-CH_2R^3$;

$R^3$ is independently alkyl;
$ALK^2$ is alkylene;
Z is H or $ALK^1$-CHO;
or the acid addition salt thereof.
[General Formula I]

**2.** The method of claim 1 wherein said immunosuppressive response is specific to a T cell population of lymphoid cells and antigen nonspecific.

**3.** The method of claim 2 wherein said immunosuppressive response is a decrease in the proliferation of said T cell population.

**4.** The method of claim 3 wherein said immunosuppressive response is the selective suppression of the proliferation of cells selected from the group consisting essentially of helper T cells and cytotoxic T cells.

**5.** The method of claim 4 wherein $ALK^1$ of General Formula I is straight or branched chain alkylene of 1 to 6 carbons; $R^3$ is straight or branched chain alkyl of 1 to 6 carbons; and $ALK^2$ is straight or branched chain alkylene of 1 to 8 carbons, and Z is $ALK^1$-CHO.

**6.** The method of claim 5 wherein $ALK^1$ is straight or branched chain alkylene of 1 to 4 carbon atoms, $R^2$ is hydrogen and $ALK^2$ is straight or branched chain alkylene of 1 to 4 carbon atoms.

**7.** The method of claim 6 wherein $ALK^1$ is alkylene of 1 to 2 carbon atoms, and $ALK^2$ is straight or branched alkylene of 1 to 3 carbon atoms.

**8.** The method of claim 7 wherein said compound of General Formula I is spermine dialdehyde.

**9.** The method of claim 3 wherein said immunosuppressive response is substantially irreversible.

**10.** The method of claim 1 wherein said living cells are selected from the group consisting essentially of bone marrow cells, spleen cells, and peripheral lymphocytes, or any combination thereof.

**11.** An <u>ex vivo</u> method of treatment of bone marrow extracts from a living organism to suppress proliferation of T cell population in said extract, which comprises administering to said extract in substantially pure form and in an amount sufficient to suppress said T cell proliferation, a compound of General Formula I:

$$OCH - ALK^1 - NR^2 - CH_2 - ALK^2 - CH_2 - NR^2 - Z$$

wherein
$ALK^1$ is independently alkylene;
$R^2$ is independently hydrogen or $-CH_2R^3$;
$R^3$ is independently alkyl;
$ALK^2$ is alkylene;
Z is H or $ALK^1$-CHO;
or the acid addition salt thereof.
[General Formula I]

**12.** A dialdehyde of the following formula:

$$OCH\text{-}ALK^1\text{-}NR^2\text{-}CH_2\text{-}ALK^2\text{-}CH_2\text{-}NR^2\text{-}ALK^1\text{-}CHO$$

wherein
$ALK^1$ is independently alkylene;
$R^2$ is independently hydrogen or $-CH_2R^3$;
$R^3$ is independently alkyl;
$ALK^2$ is alkylene;

or the addition salt thereof, providing however, that if $ALK^1$ and $ALK^2$ are both ethylene, then $R^2$ is $CH_2R^3$.

**13.** The dialdehyde of claim 12 wherein,

| | |
|---|---|
| $ALK^1$ | is independently straight or branched chain alkylene of 1 to 6 carbons; |
| $R^3$ | is independently straight or branched chain alkyl of 1 to 6 carbons; and |
| $ALK^2$ | is independently straight or branched chain alkylene of 1 to 8 carbons. |

**14.** A compound of General Formula I, as recited in claim 1, for use in therapy.

**15.** A substantially pure compound of General Formula I, as recited in claim 1, for use in therapy.

**16.** A compound according to claim 14 or claim 15 wherein $R^2$ is hydrogen.

**17.** A pharmaceutical composition comprising a compound of General Formula I, as recited in claim 1, or a mixture of such compounds, together with a pharmaceutically acceptable carrier, said composition containing less than 5% by weight, and preferably less than 1% by weight of other oxidized polyamines.

**18.** A composition according to claim 17 for use in therapy.

**19.** A method of making a composition according to claim 17, comprising combining a substantially pure compound of General Formula I, as recited in claim 1, with a pharmaceutically acceptable carrier.

**20.** The use of a compound of General Formula I, as recited in claim 1, in the manufacture of a medicament for inducing an immunosuppressive response in living cells in vivo.

**21.** The use of a compound according to any of claims 12 to 14 in the manufacture of a composition for suppressing an immunological graft tissue vs. host reaction in an individual for whom such therapy is indicated.

**Patentansprüche**

**1.** Verfahren zum Induzieren einer immunsuppressiven Reaktion in lebenden Zellen in vitro, umfassend, daß man den Zellen eine Verbindung der allgemeinen Formel I:

$$OCH - ALK^1 - NR^2 - CH_2 - ALK^2 - CH_2 - NR^2 - Z,$$

in der

| | |
|---|---|
| $ALK^1$ | unabhängig Alkylen ist; |
| $R^2$ | unabhängig Wasserstoff oder $-CH_2R^3$ ist; |
| $R^3$ | unabhängig Alkyl ist; |
| $ALK^2$ | gleich Alkylen ist; |
| Z | gleich H oder $ALK^1$-CHO ist; |

oder ihr Säureadditionssalz,
[Allgemeine Formel I]
in einer im wesentlichen reinen Form und in einer zum Induzieren der Reaktion wirksamen Menge verabreicht.

**2.** Verfahren nach Anspruch 1, in dem die immunsuppressive Reaktion auf eine T-Zellen-Population von lymphoiden Zellen spezifisch und auf Antigen nichtspezifisch ist.

**3.** Verfahren nach Anspruch 2, in dem die immunsuppressive Reaktion eine Abnahme in der Proliferation der T-Zellen-Population ist.

**4.** Verfahren nach Anspruch 3, in dem die immunsuppressive Reaktion die selektive Suppression der Proliferation von Zellen ist, die ausgewählt sind aus der Gruppe bestehend im wesentlichen aus T-Helfer-Zellen und zytotoxischen T-Zellen.

5. Verfahren nach Anspruch 4, in dem $ALK^1$ der allgemeinen Formel I ein gerad- oder verzweigtkettiges Alkylen mit 1 bis 6 Kohlenstoffatomen ist; $R^3$ ein gerad- oder verzweigtkettiges Alkyl mit 1 bis 6 Kohlenstoffatomen ist; und $ALK^2$ ein gerad- oder verzweigtkettiges Alkylen mit 1 bis 8 Kohlenstoffatomen ist und Z gleich $ALK^1$-CHO ist.

6. Verfahren nach Anspruch 5, in dem $ALK^1$ ein gerad- oder verzweigtkettiges Alkylen mit 1 bis 4 Kohlenstoffatomen ist, $R^2$ gleich Wasserstoff ist und $ALK^2$ ein gerad- oder verzweigtkettiges Alkylen mit 1 bis 4 Kohlenstoffatomen ist.

7. Verfahren nach Anspruch 6, in dem $ALK^1$ ein Alkylen mit 1 bis 2 Kohlenstoffatomen ist und $ALK^2$ ein gerad- oder verzweigtkettiges Alkylen mit 1 bis 3 Kohlenstoffatomen ist.

8. Verfahren nach Anspruch 7, in dem die Verbindung der allgemeinen Formel I Spermindialdehyd ist.

9. Verfahren nach Anspruch 3, in dem die immunsuppressive Reaktion im wesentlichen irreversibel ist.

10. Verfahren nach Anspruch 1, in dem die lebenden Zellen ausgewählt sind aus der Gruppe bestehend im wesentlichen aus Knochenmarkzellen, Milzzellen und peripheren Lymphozyten oder jeder beliebigen Kombination davon.

11. Ex vivo-Verfahren zur Behandlung von Knochenmarkextrakten aus einem lebenden Organismus zur Proliferationssuppression der T-Zellen-Population in dem Extrakt, umfassend, daß man dem Extrakt eine Verbindung der allgemeinen Formel I:

$$OCH - ALK^1 - NR^2 - CH_2 - ALK^2 - CH_2 - NR^2 - Z,$$

in der
- $ALK^1$ unabhängig Alkylen ist;
- $R^2$ unabhängig Wasserstoff oder $-CH_2R^3$ ist;
- $R^3$ unabhängig Alkyl ist;
- $ALK^2$ gleich Alkylen ist;
- Z gleich H oder $ALK^1$-CHO ist;

oder ihr Säureadditionssalz,
[Allgemeine Formel I]
in einer im wesentlichen reinen Form und in einer Menge, die ausreicht, um die T-Zellen-Proliferation zu unterdrücken, verabreicht.

12. Dialdehyd der folgenden Formel:

$$OCH-ALK^1-NR^2-CH_2-ALK^2-CH_2-NR^2-ALK^1-CHO,$$

in der
- $ALK^1$ unabhängig Alkylen ist;
- $R^2$ unabhängig Wasserstoff oder $-CH_2R^3$ ist;
- $R^3$ unabhängig Alkyl ist;
- $ALK^2$ gleich Alkylen ist;

oder ihr Additionssalz, mit der Maßgabe, daß, wenn $ALK^1$ und $ALK^2$ beide Ethylen sind, $R^2$ gleich $CH_2R^3$ ist.

13. Dialdehyd nach Anspruch 12, wobei
- $ALK^1$ unabhängig ein gerad- oder verzweigtkettiges Alkylen mit 1 bis 6 Kohlenstoffatomen ist;
- $R^3$ unabhängig ein gerad- oder verzweigtkettiger Alkylrest mit 1 bis 6 Kohlenstoffatomen ist; und
- $ALK^2$ unabhängig ein gerad- oder verzweigtkettiges Alkylen mit 1 bis 8 Kohlenstoffatomen ist.

14. Verbindung der allgemeinen Formel I nach Anspruch 1 zur Verwendung in der Therapie.

**15.** Im wesentlichen reine Verbindung der allgemeinen Formel I nach Anspruch 1 zur Verwendung in der Therapie.

**16.** Verbindung nach Anspruch 14 oder Anspruch 15, in der $R^2$ gleich Wasserstoff ist.

**17.** Pharmazeutische Zusammensetzung, umfassend eine Verbindung der allgemeinen Formel I nach Anspruch 1 oder ein Gemisch solcher Verbindungen, zusammen mit einem pharmazeutisch verträglichen Träger, wobei die Zusammensetzung weniger als 5 Gew.-%, und vorzugsweise weniger als 1 Gew.-%, andere oxidierte Polyamine enthält.

**18.** Zusammensetzung nach Anspruch 17 zur Verwendung in der Therapie.

**19.** Verfahren zum Herstellen einer Zusammensetzung nach Anspruch 17, umfassend das Vereinigen einer im wesentlichen reinen Verbindung der allgemeinen Formel I nach Anspruch 1 mit einem pharmazeutisch verträglichen Träger.

**20.** Verwendung einer Verbindung der allgemeinen Formel I nach Anspruch 1 bei der Herstellung eines Medikaments zum Induzieren einer immunsuppressiven Reaktion in lebenden Zellen in vivo.

**21.** Verwendung einer Verbindung nach einem der Ansprüche 12 bis 14 bei der Herstellung einer Zusammensetzung zur Suppression einer immunologischen Reaktion von Transplantatgewebe gegen Wirt in einem Individuum, für das eine solche Therapie angezeigt ist.

**Revendications**

**1.** Un procédé pour provoquer une réponse immunosuppressive dans des cellules vivantes in vitro, qui consiste à administrer auxdites cellules, sous sa forme pratiquement pure et en une quantité efficace pour provoquer ladite réponse, un composé de la formule générale I :

$$OCH - ALK^1 - NR^2 - CH_2 - ALK^2 - CH_2 - NR^2 - Z$$

dans laquelle

| | |
|---|---|
| $ALK^1$ | représente, de façon indépendante, un alcylène ; |
| $R^2$ | représente, de façon indépendante, de l'hydrogène ou $-CH_2R^3$ ; |
| $R^3$ | représente, de façon indépendante, un alkyle ; $ALK^2$ est un alcylène ; |
| Z | est H ou $ALK^1$-CHO ; |

ou un de ses sels d'addition d'acide.

[Formule générale I]

**2.** Le procédé de la revendication 1, dans lequel ladite réponse immunosuppressive est spécifique d'une population de cellules T de cellules lymphoïdes et non-antigène spécifique.

**3.** Le procédé de la revendication 2, dans lequel ladite réponse immunosuppressive est une diminution de la prolifération de ladite population de cellules T.

**4.** Le procédé de la revendication 3, dans lequel ladite réponse immunosuppressive est la suppression sélective de la prolifération de cellules choisies dans le groupe constitué essentiellement de cellules auxiliaires T et de cellules cytotoxiques T.

**5.** Le procédé de la revendication 4, dans lequel $ALK^1$ de la formule générale I est un alcylène, à chaîne droite ou ramifiée, de 1 à 6 carbones ; $R^3$ est un alkyle à chaîne droite ou ramifiée de 1 à 6 carbones ; et $ALK^2$ est un alcylène, à chaîne droite ou ramifiée de 1 à 8 carbones et Z est $ALK^1$-CHO.

**6.** Le procédé de la revendication 5, dans lequel $ALK^1$ est un alcylène, à chaîne droite ou ramifiée, de 1 à 4 atomes de carbone, $R^2$ est de l'hydrogène et $ALK^2$ est un alcylène, à chaîne droite ou ramifiée, de 1 à 4 atomes de carbone.

**7.** Le procédé de la revendication 6, dans lequel $ALK^1$ est un alcylène de 1 à 2 atomes de carbone, et $ALK^2$ est un alcylène, à chaîne droite ou ramifiée, de 1 à 3 atomes de carbone.

**8.** Le procédé de la revendication 7, dans lequel ledit composé de formule générale I est le spermine dialdéhyde.

**9.** Le procédé de la revendication 3, dans lequel ladite réponse immunosuppressive est pratiquement irréversible.

**10.** Le procédé de la revendication 1, dans lequel lesdites cellules vivantes sont choisies dans le groupe constitué essentiellement des cellules de la moelle osseuse, des cellules du pancréas et des lymphocytes périphériques, ou bien une combinaison quelconque de celles-ci.

**11.** Un procédé de traitement ex vivo d'extraits de moelle osseuse provenant d'un organisme vivant afin de supprimer la prolifération d'une population de cellules T dans les extraits, qui consiste à administrer auxdits extraits, sous une forme pratiquement pure et en une quantité suffisante pour supprimer ladite prolifération des cellules T, un composé de la formule générale I :

$$OCH - ALK^1 - NR^2 - CH_2 - ALK^2 - CH_2 - NR^2 - Z$$

dans laquelle
- $ALK^1$ représente, de façon indépendante un alcylène ;
- $R^2$ représente, de façon indépendante, de l'hydrogène ou $-CH_2R^3$ ;
- $R^3$ représente, de façon indépendante, un alkyle ;
- $ALK^2$ est un alcylène ;
- Z est H ou $ALK^1$-CHO ;

ou un de ses sels d'addition d'acide.
[Formule générale I]

**12.** Un dialdéhyde de la formule suivante :

$$OCH - ALK^1 - NR^2 - CH_2 - ALK^2 - CH_2 - NR^2 - ALK^1 - CHO$$

dans laquelle
- $ALK^1$ représente, de façon indépendante, un alcylène ;
- $R^2$ représente, de façon indépendante, de l'hydrogène ou $-CH_2R^3$ ;
- $R^3$ représente, de façon indépendante, un alkyle ;
- $ALK^2$ est un alcylène ;

ou bien un de ses sels d'addition, sous la condition que toutefois si $ALK^1$ et $ALK^2$ sont tous les deux de l'éthylène, alors $R^2$ est $CH_2R^3$.

**13.** Le dialdéhyde de la revendication 12 dans lequel, $ALK^1$ représente, de façon indépendante, un alcylène, à
chaîne droite ou ramifiée, de 1 à 6 carbones ; $R^3$ représente, de façon indépendante, un alkyle, à chaîne droite ou ramifiée, de 1 à 6 carbones ; et
$ALK^2$ représente, de façon indépendante, un alcylène, à chaîne droite ou ramifiée, de 1 à 8 carbones.

**14.** Un composé de la formule générale I, tel que spécifié dans la revendication 1, destiné à être utilisé en thérapie.

**15.** Un composé pratiquement pur de formule générale I, tel que spécifié dans la revendication 1, destiné à être utilisé en thérapie.

**16.** Un composé selon la revendication 14 ou la revendication 15, dans lequel $R^2$ est de l'hydrogène.

**17.** Une composition pharmaceutique comportant un composé de formule générale I, tel que spécifié dans la revendication 1, ou bien un mélange de ces composés, conjointement avec un support pharmaceutiquement acceptable, ladite composition renfermant moins de 5% en poids, et de préférence moins de

1% en poids d'autres polyamines oxydées.

**18.** Une composition selon la revendication 17, destinée à être utilisée en thérapie.

**19.** Un procédé de préparation d'une composition selon la revendication 17, procédé selon lequel on combine un composé pratiquement pur de formule générale I, tel que spécifié dans la revendication 1, avec un support pharmaceutiquement acceptable.

**20.** L'utilisation d'un composé de la formule générale I, tel que spécifié dans la revendication 1, dans la fabrication d'un médicament pour provoquer une réponse immunosuppressive dans les cellules vivantes in vivo.

**21.** L'utilisation d'un composé selon l'une quelconque des revendications 12 à 14 pour la préparation d'une composition destinée à supprimer une réaction immunologique de l'hôte à l'égard d'un tissu greffé dans un individu pour lequel une telle thérapie est indiquée.

FIG-1 IN VITRO SUPPRESSION OF HUMAN T & B LYMPHOCYTES BY SPERMINE DIALDEHYDE

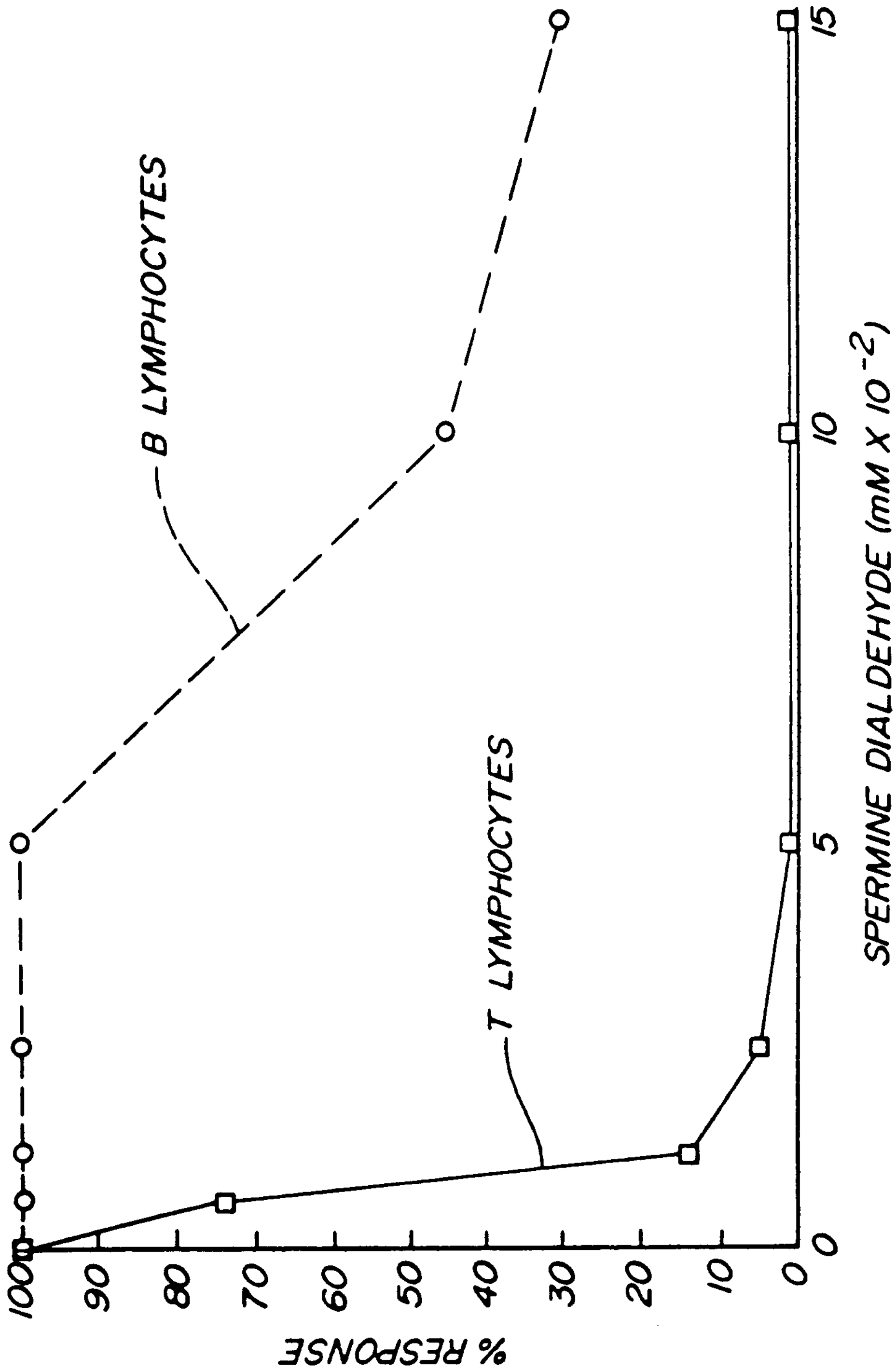

FIG-2 IN VITRO SUPPRESSION MEDIATED BY SPERMIDINE DIALDEHYDE

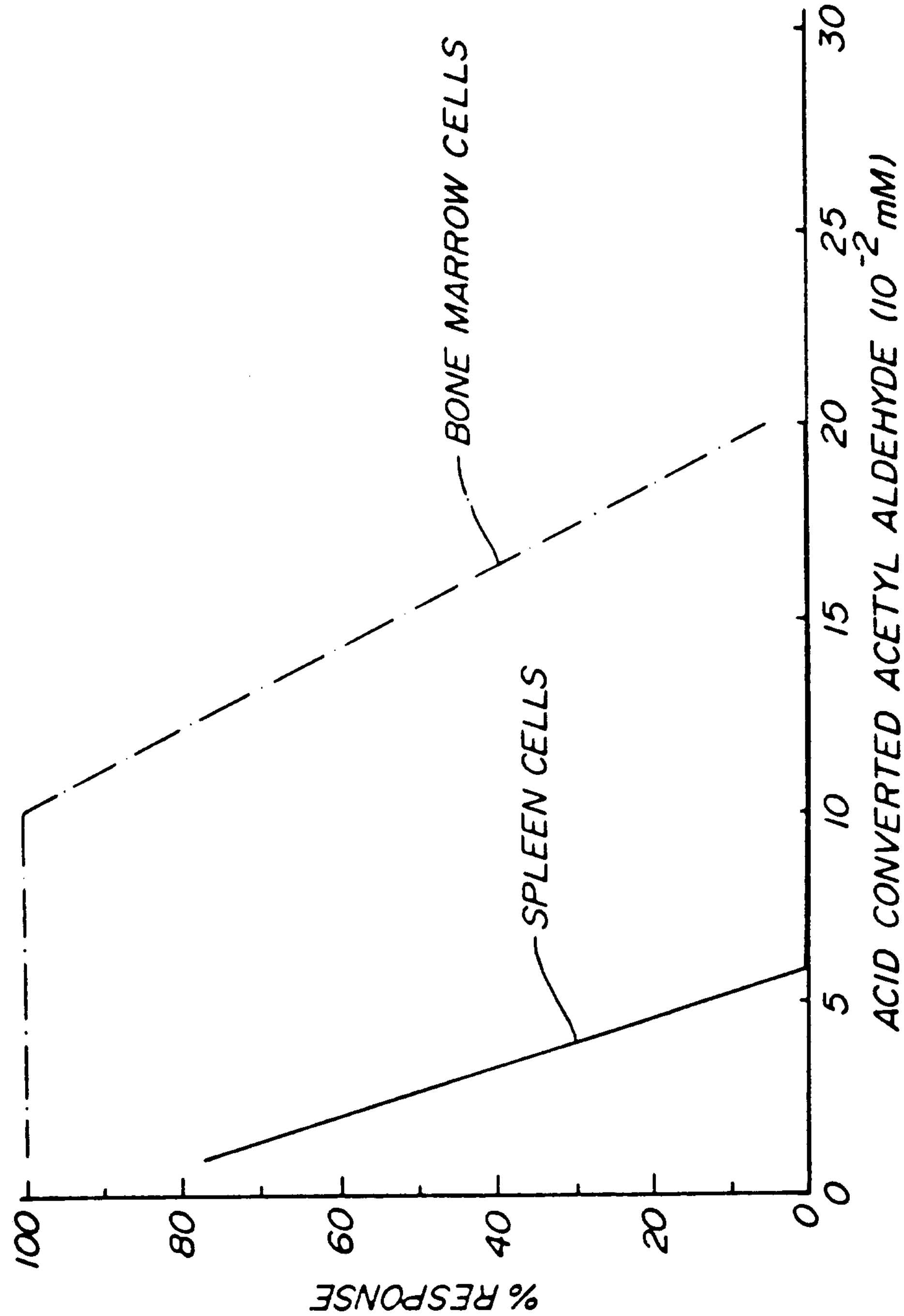

FIG-3 EFFECT OF SPERMINE DIALDEHYDE ON SURVIVAL AFTER ALLOGENIC BMT IN MICE (C57BL/6-AKR)

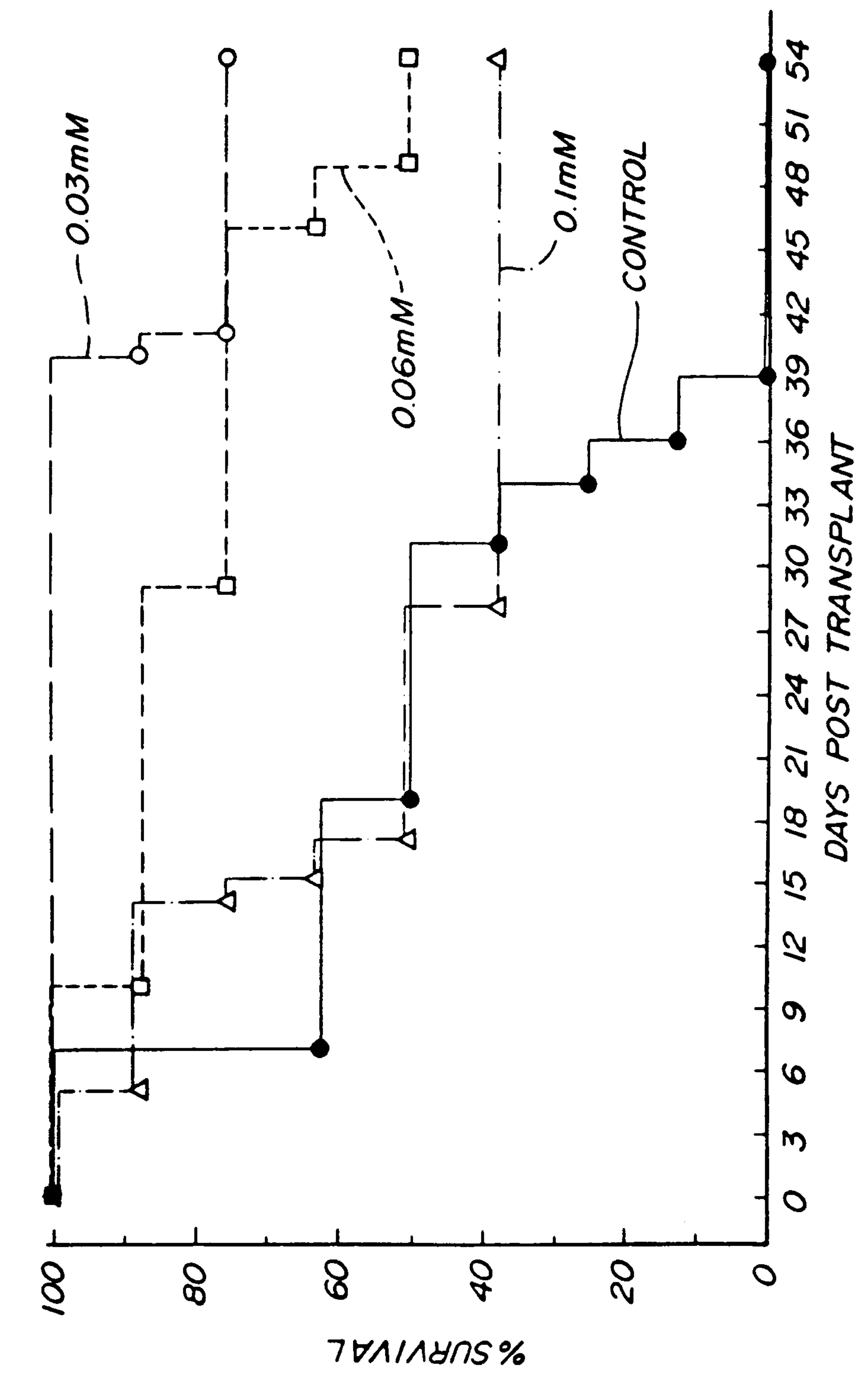

FIG-4 EFFECT OF SPERMINE DIALDEHYDE ON GRAFT VS HOST REACTION (POPLITEAL LYMPH NODE ASSAY)

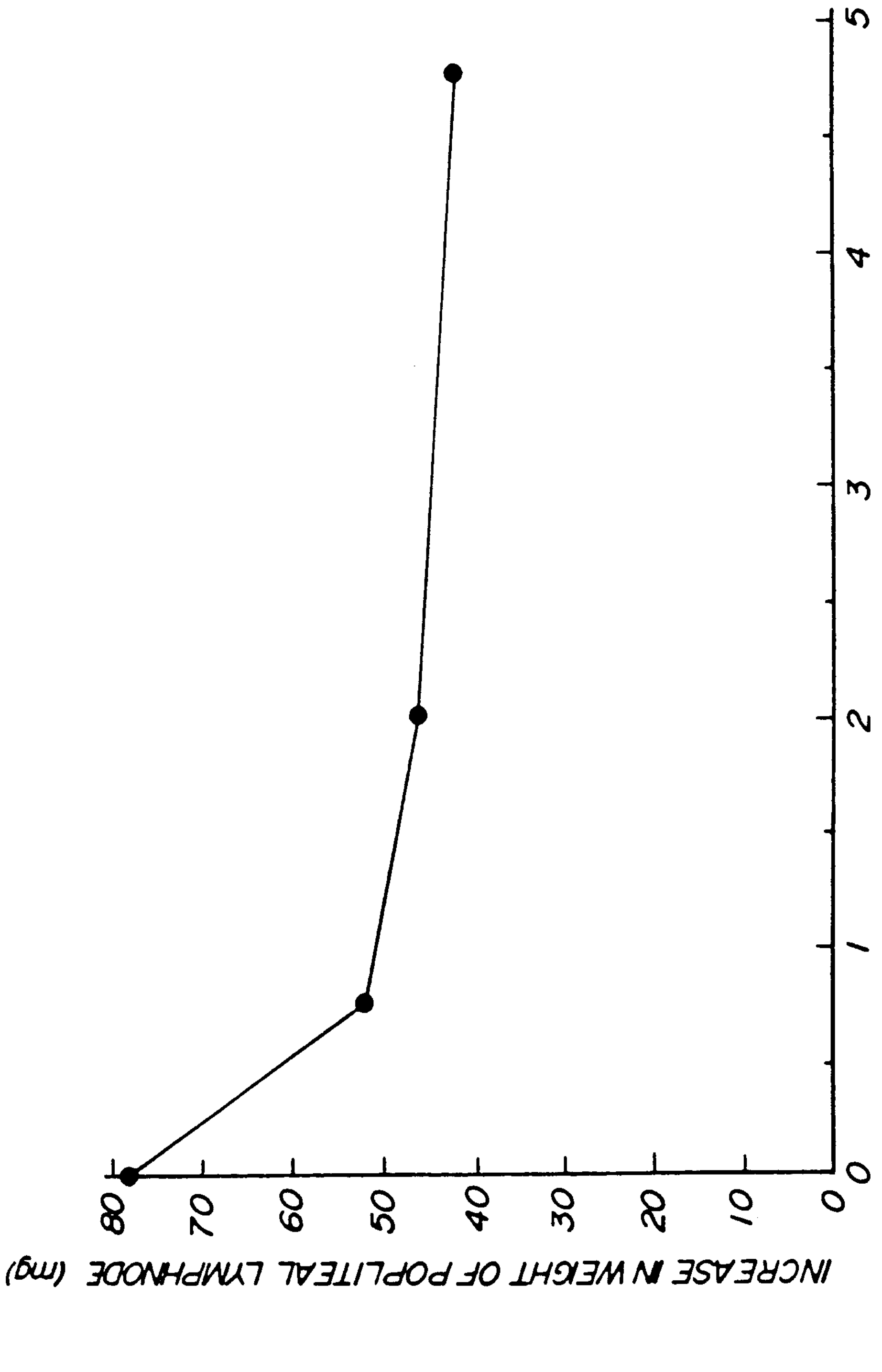

FIG-5 IN VIVO EFFECT OF SPERMINE DIALDEHYDE ON CELL MEDIATED LYMPHOLYSIS IN MICE

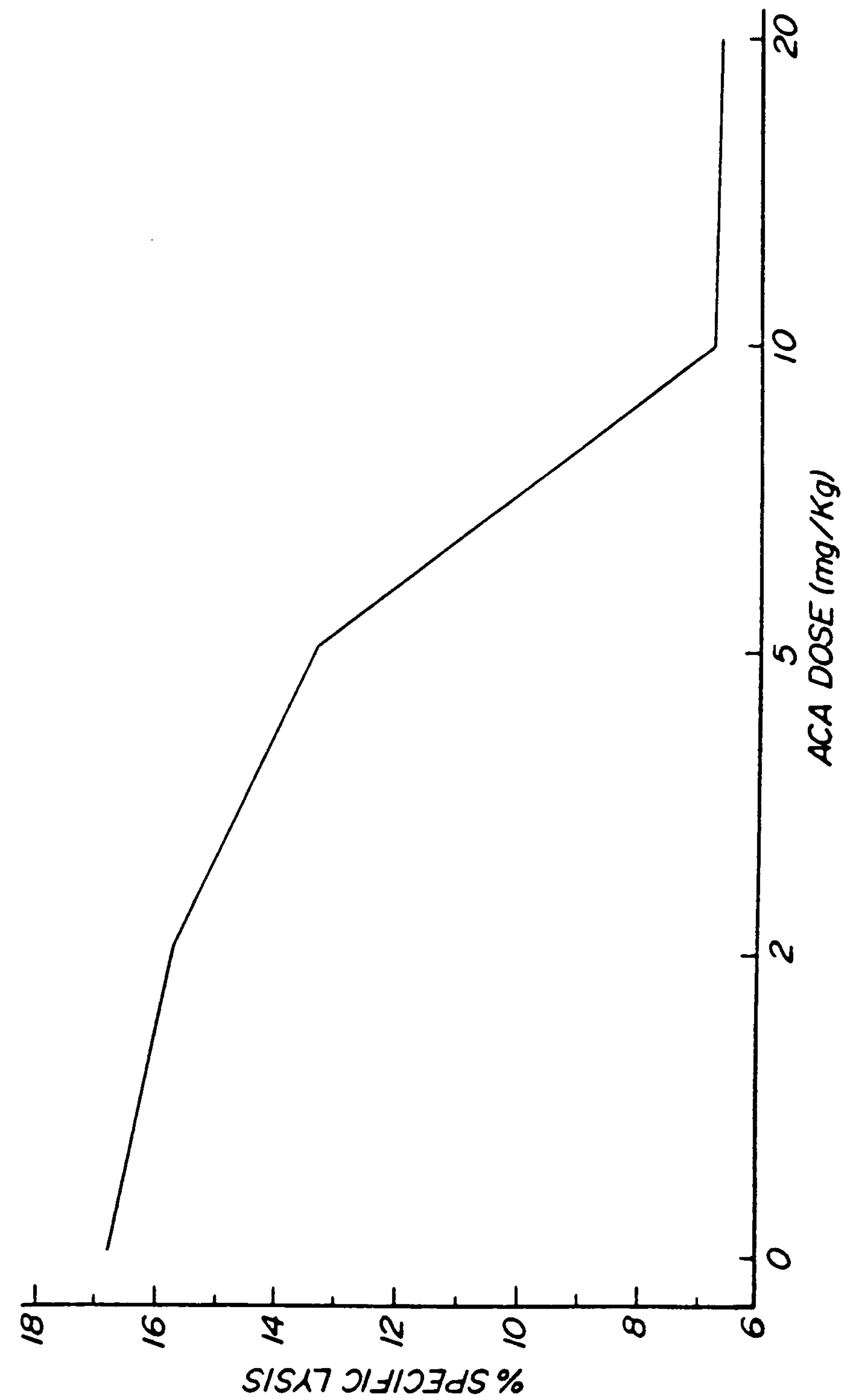

FIG-6 PROLONGATION OF SKIN GRAFT SURVIVAL BY SPERMINE DIALDEHYDE

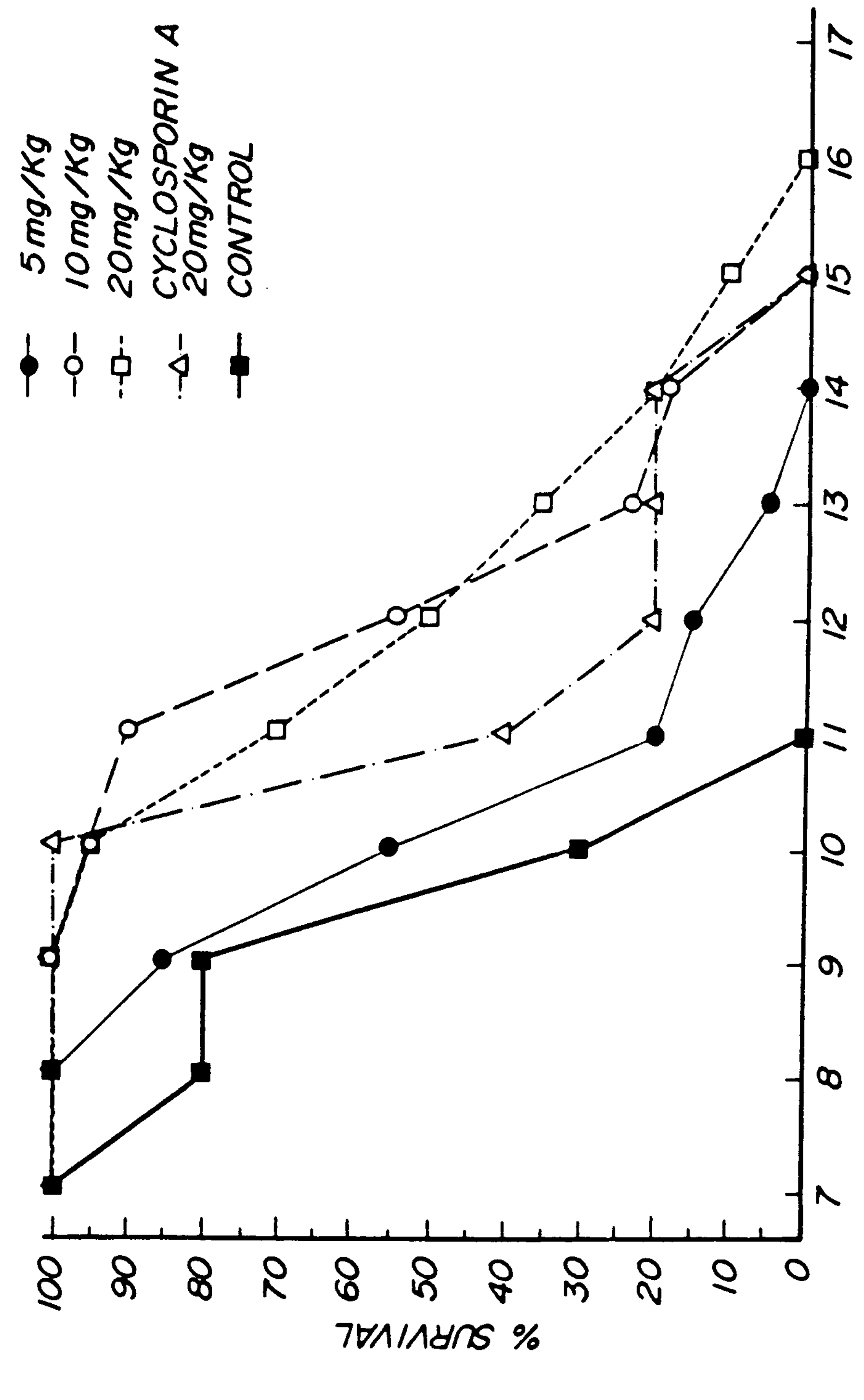

FIG-7 SUPPRESSION OF DELAYED-TYPE HYPERSENSITIVITY BY SPERMINE DIALDEHYDE

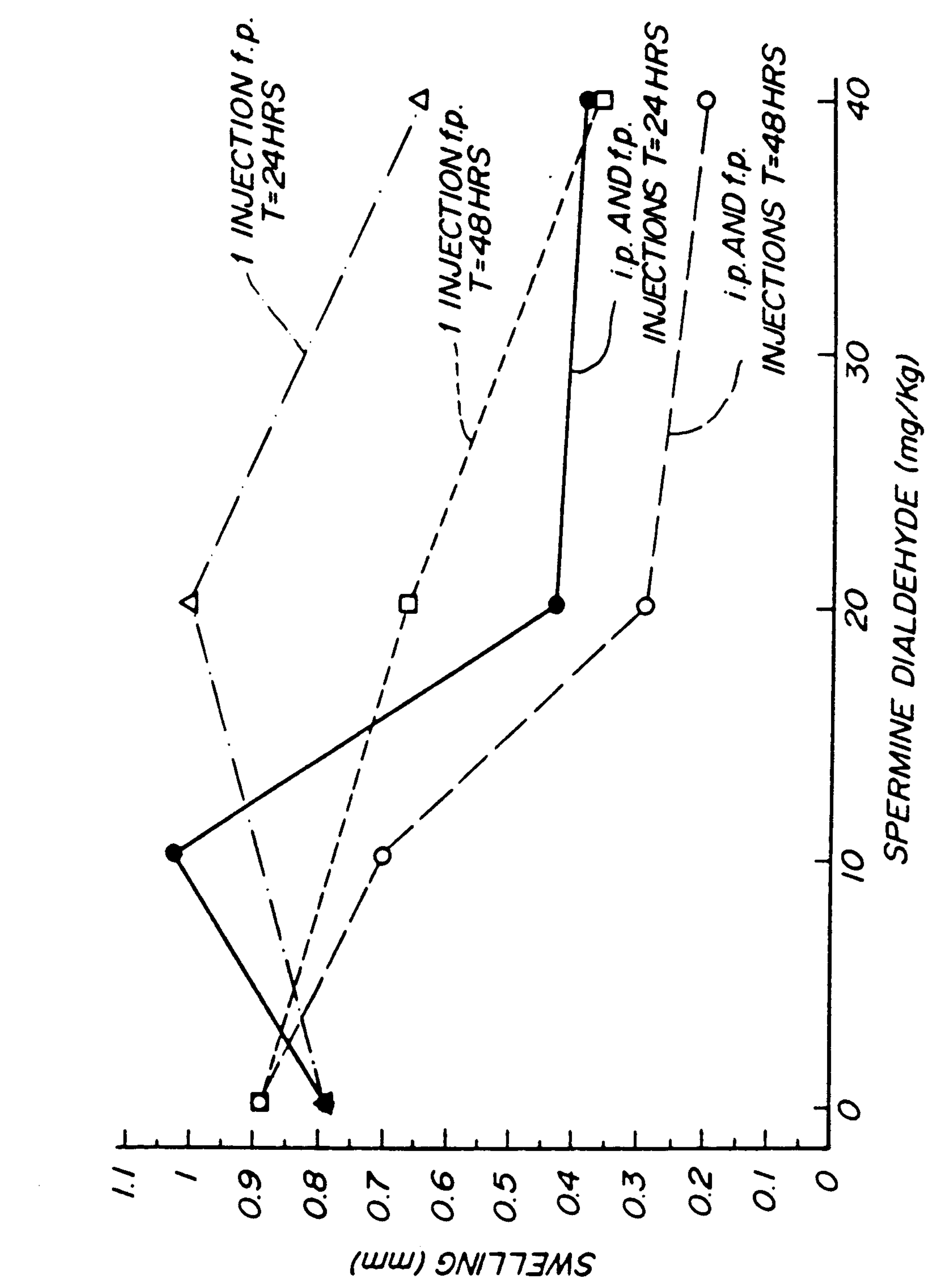

FIG-8

EFFECT OF SDA AND BUTYL DIALDEHYDE ON THE GROWTH OF MITOGEN STIMULATED SPLENIC T CELLS

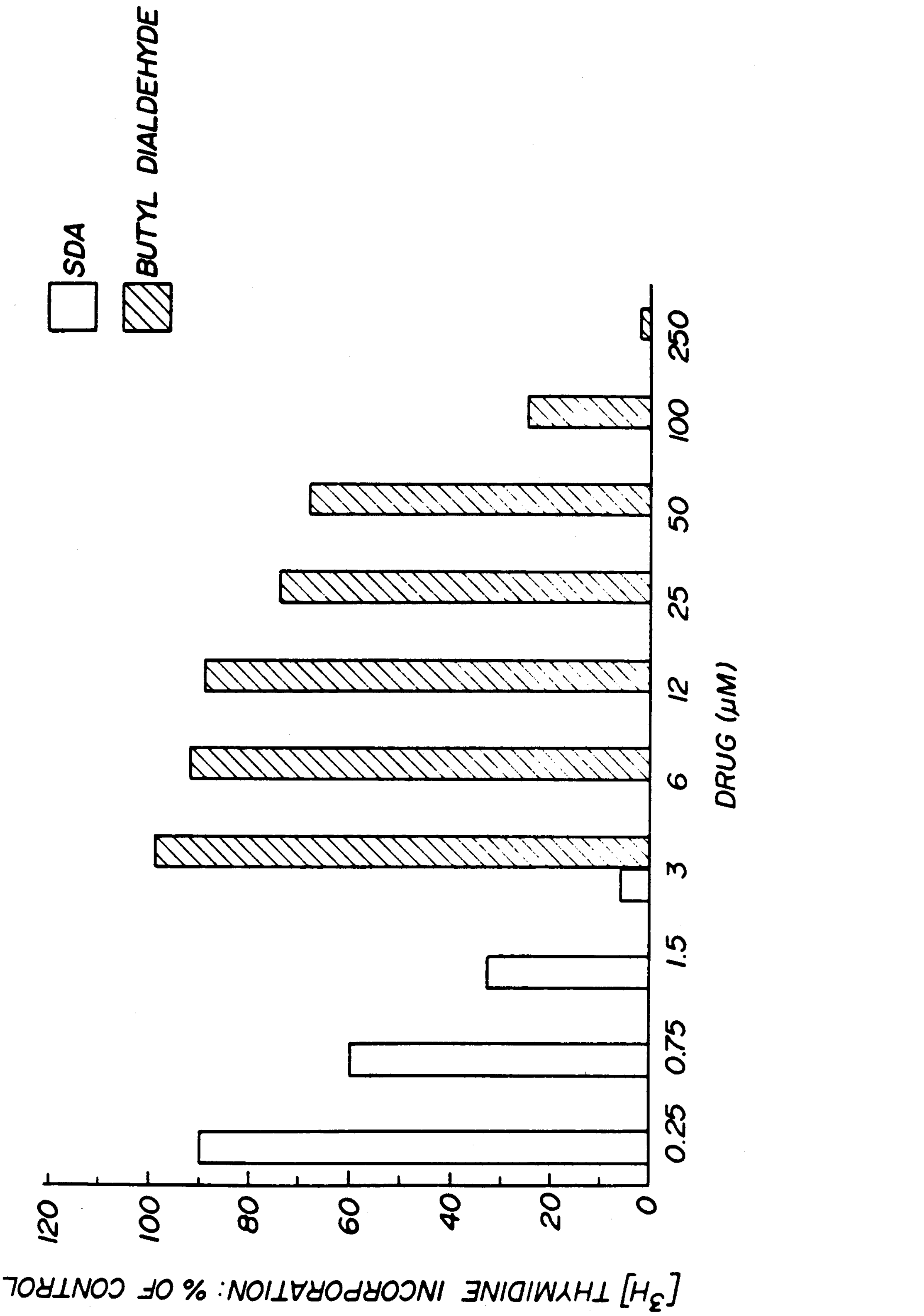

FIG-9 AdoMetDC IN L1210 CELLS

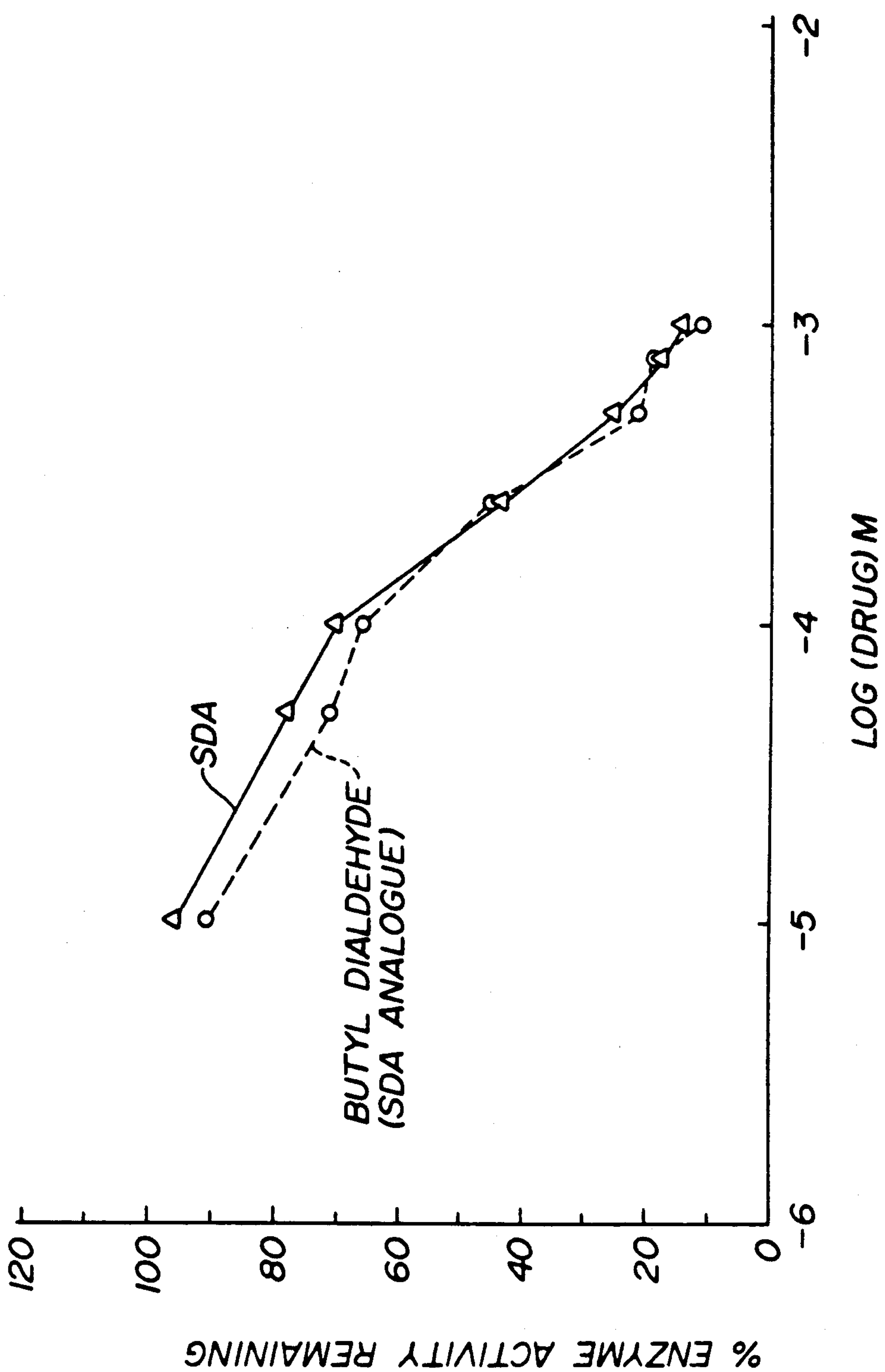

FIG-10 POLYAMINE BIOSYNTHETIC ENZYMES IN L1210 CELLS